(19) Europäisches Patentamt
European Patent Office
Office européen des brevets

(11) EP 4 280 877 B1

(12) FASCICULE DE BREVET EUROPEEN

(45) Date de publication et mention de la délivrance du brevet:
**15.07.2026 Bulletin 2026/29**

(21) Numéro de dépôt: **22701350.5**

(22) Date de dépôt: **17.01.2022**

(51) Classification Internationale des Brevets (IPC):
***A01N 43/16*** *(2006.01)* ***A01P 17/00*** *(2006.01)*
***A01P 19/00*** *(2006.01)* ***C07D 311/22*** *(2006.01)*

(52) Classification Coopérative des Brevets (CPC):
(C-Sets disponibles)
**A01P 17/00; A01N 43/16; A01P 19/00;**
**C07D 311/22;** Y02A 50/30 (Cont.)

(86) Numéro de dépôt international:
**PCT/EP2022/050882**

(87) Numéro de publication internationale:
**WO 2022/157118 (28.07.2022 Gazette 2022/30)**

(54) **DÉRIVÉS DE CHROMONE COMME ATTRACTANTS ET RÉPULSIFS D'INVERTÉBRÉS PIQUEURS HÉMATOPHAGES**

CHROMONDERIVATE ALS LOCK- OR ABSCHRECKMITTEL BLUTSAUGENDER STECHENDE WIRBELLOSE

CHROMONE DERIVATIVES AS ATTRACTANTS OR REPELLENTS FOR STINGING HAEMATOPHAGOUS INVERTEBRATES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.01.2021 FR 2100502**

(43) Date de publication de la demande:
**29.11.2023 Bulletin 2023/48**

(73) Titulaires:
- **Centre National de la Recherche Scientifique**
**75016 Paris (FR)**
- **CPE Lyon**
**69616 Villeurbanne Cedex (FR)**
- **INSTITUT NATIONAL DE LA SANTE ET DE LA RECHERCHE MEDICALE (INSERM)**
**75013 Paris (FR)**
- **Institut de Recherche pour le Développement**
**13572 Marseille Cedex 02 (FR)**
- **Université Claude Bernard Lyon I**
**69622 Villeurbanne Cedex (FR)**
- **Universite De La Reunion**
**97400 Saint Denis (FR)**
- **Tropical Biocontrol**
**97490 Saint-Denis (FR)**

(72) Inventeurs:
- **ANDRIANJAFY, Mbolatiana Tovo**
**Antananarivo (MG)**
- **VESTALYS, Voahangy**
**Antananarivo (MG)**
- **LEMAIRE, Marc**
**69100 Villeurbanne (FR)**
- **MAVINGUI, Patrick**
**97490 Sainte Clotilde (FR)**
- **RAMIHARIMANANA, Fenia Diane**
**Antananarivo (MG)**
- **LAMARE, Saholy**
**97400 Saint-Denis (FR)**

(74) Mandataire: **Santarelli**
**Tour Trinity**
**1 bis Place de la Défense**
**92400 Courbevoie (FR)**

(56) Documents cités:
**US-A1- 2012 329 832**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **BOGGU PULLA REDDY ET AL: "Discovery of novel 3-(hydroxyalkoxy)-2-alkylchromen-4-one analogs as interleukin-5 inhibitors", vol. 139, 1 October 2017 (2017-10-01), AMSTERDAM, NL, pages 290 - 304, XP055838568, ISSN: 0223-5234, Retrieved from the Internet <URL:https://www.sciencedirect.com/science/article/pii/S0223523417305913/pdfft?md5=23282c538d1a4b591de4077dcf2d144d&pid=1-s2.0-S0223523417305913-main.pdf> DOI: 10.1016/j.ejmech.2017.07.069**

Remarques:

Le dossier contient des informations techniques présentées postérieurement au dépôt de la demande et ne figurant pas dans le présent fascicule.

(52) Classification Coopérative des Brevets (CPC): (Cont.)

C-Sets
**A01N 43/16, A01N 43/16**

## Description

La présente demande a pour objet des dérivés de chromone comme attractants et répulsifs d'invertébrés piqueurs hématophages, notamment de moustiques. La présente demande a également pour objet des compositions attractives ou répulsives pour des invertébrés piqueurs hématophages.

Les insectes jouent un rôle important dans l'équilibre écologique. Ils sont responsables de la plus grande partie de la pollinisation des plantes et sont un important échelon de la chaîne alimentaire. Mais certains insectes sont nuisibles pour les cultures (les ravageurs) voire dangereux puisque vecteurs d'agents infectieux à l'homme et aux animaux. Par exemple, les moustiques sont responsables de la majorité des cas de transmission des agents pathogènes à l'origine des maladies infectieuses. Notamment l'espèce *Aedes albopictus* qui est vecteur de plusieurs arbovirus tels que la Dengue, le Chikungunya, la Fièvre Jaune et le Zika. La méthode de lutte la plus utilisée pour contrôler les populations de moustiques est l'application d'insecticides qui peuvent être efficaces, mais posent des problèmes de pollutions importantes à cause de leurs toxicités, de leurs larges spectres d'action qui peuvent tuer les populations non cibles (insectes utiles et auxiliaires). De plus, l'apparition de résistance aux insecticides au sein des espèces vectrices rend globalement l'approche peu compatible avec un développement durable. L'utilisation de répulsifs pour protéger les populations exposées ou d'attractants pour la construction de pièges spécifiques apparait de plus en plus comme des compléments et/ou alternatives à l'utilisation d'insecticides. Il existe déjà des répulsifs relativement efficaces comme le DEET, la picaridine, le PMD, mais ces molécules présentent souvent des effets secondaires (irritations oculaire et cutanée, céphalées, problèmes respiratoires) et/ou des efficacités limitées du point de vue temps de protection. Il existe aussi des attractifs connus (acide isovalérique, 3-octenol ou dioxyde de carbone...) mais ceux-ci ne possèdent pas de sélectivité vis-à-vis des moustiques et ont des durées d'activités faibles (Thèse Andrianjafy. 2018, 119,120p - « Ecologie chimique, une alternative de lutte pour le contrôle des moustiques (Diptera: Culicidae) vecteurs de maladies : bioessai, synthèse et évaluation sur terrain des répulsifs et attractifs ». Thèse de doctorat à l'école doctorale Valorisation de ressources naturelles renouvelables de l'Université d'Antananarivo). Le problème de la durée d'activité est important et ce paramètre est lié à la forte volatilité des produits utilisés qui sont des liquides à forte tension de vapeurs. Les coumarines sont une famille de molécules à faible tension de vapeur avec soit des propriétés répulsives, soit des propriétés attractantes (Thèse Andrianjafy.2018, 110-122p ; Andrianjafy *et al.* 2018). Ces molécules sont efficaces mais pour obtenir des effets répulsifs ou attractifs suffisamment élevés et sélectifs, il est généralement nécessaire d'utiliser des mélanges d'attractifs ou de répulsifs (Thèse Andrianjafy 2018, 120-121p ; Andrianjafy MT, Ravaomanarivo LH, Vestalys Ramanandraibe V, Rakotomanga MF, Mavingui P, Lemaire M (2018) Synthesis, bioassays and field evaluation of hydroxycoumarins and their alkyl derivatives as repellents or kairomones for Aedes albopictus Skuse (Diptera: Culicidae). J Chem Ecol 44(3):299-311 ; Becker NM, Zgomba DP, and Ludwig M (1995) Comparison of carbon dioxide, octanol and o a houst-odour as mosquito attractants in the Upper Valley, Germany. Med Vet Entomol 9: 377-380 ; Hall DR, Beevor PS, Cork A, Nesbitt BF and Vale GA (1984) 1-octen-ol: a potent olfactory stimulant and attractant for tsetse isolated from cattle odours. Insect Sci Appl 5:335-339 ; Shone SM, Ferrao PN, Lesser CR, Glass GE and Norris DE (2003) Evaluation of carbon dioxide and 1-octen-3-ol-baited centers for disease control Fay-Prince traps to collect Aedes albopictus. J Am Mosq Control Assoc 19:445-447 ; Govere J, Durrheim DN, Du Toit N, Hunt RH (2000) Local plants as repellents against Anopheles arabiensis, in Mpumalanga Province, South Africa. Cent Afr J Med 46(8):213-6 ; Barnard DR (2004) Laboratory evaluation of mosquito repellents against Aedes albopictus, Culex nigripalpus, and Ochlerotatus triseriatus (Diptera: Culicidae). Journal of Med Entomol 41:726-730). D'ailleurs dans la nature, les insectes utilisent aussi des mélanges de molécules (composés sémiochimiques) pour interagir avec leur environnement, entre eux et avec les autres êtres vivants. Il est donc nécessaire de rechercher de nouvelles molécules capables d'interagir sélectivement avec les insectes dangereux afin de préparer des formulations répulsives (sprays, crèmes, moustiquaires, vêtements...) et attractives (leurres, pièges sélectifs) respectivement pour limiter le contact hôte-vecteur et pour contrôler les populations d'insectes cibles. US 2012/329832 utilise des coumarines à cet effet.

La présente invention a pour but de fournir une famille de composés présentant des propriétés attractantes ou répulsives pour des invertébrés piqueurs hématophages, notamment pour des moustiques.

La présente invention a également pour but de fournir des compositions attractives ou répulsives pour des invertébrés piqueurs hématophages, notamment pour des moustiques.

Ainsi, la présente invention concerne l'utilisation d'un composé de formule (I-1) suivante :

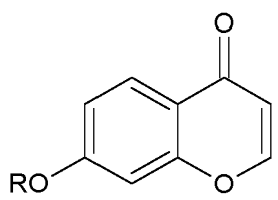

(I-1)

R représentant un groupe alkyle, linéaire ou ramifié, comprenant de 2 à 20 atomes de carbone,
ledit composé étant achiral lorsque R est un groupe linéaire ou sous forme d'un mélange racémique, d'un énantiomère (*R*) ou d'un énantiomère (*S*) lorsque R est un groupe ramifié,
comme attractif ou répulsif pour les invertébrés piqueurs hématophages.

Le terme « invertébrés piqueurs hématophages » désigne des invertébrés piqueurs qui se nourrissent de sang, notamment des insectes piqueurs s'attaquant aux êtres humains ou au cheptel ou encore aux animaux domestiques.

Selon un mode de réalisation, les invertébrés piqueurs hématophages sont choisis parmi les insectes et acariens hématophages, en particulier les insectes hématophages.

Selon un mode de réalisation préféré, les invertébrés piqueurs hématophages sont choisis dans le groupe constitué des moustiques, des ceratopogonidés, des phlébotomes, des punaises, des puces et des tiques.

Selon un mode de réalisation préféré, les invertébrés piqueurs hématophages sont choisis parmi les moustiques des genres *Aedes*, *Anopheles* et *Culex.*

Selon un mode de réalisation préféré, les invertébrés piqueurs hématophages sont des moustiques choisis dans le groupe constitué des espèces : *Aedes albopictus*, *Aedes aegypti*, *Anopheles gambiae s.l*, *Anopheles mascarensis, Anopheles funestus, Anopheles stephensi, Culex pipiens quinquefasciatus* et *Culex pipiens pipiens.*

Selon l'invention, un composé attractif pour les invertébrés piqueurs hématophages est un composé présentant des propriétés attractantes pour les invertébrés piqueurs hématophages, c'est-à-dire un composé qui attire les invertébrés piqueurs hématophages.

Selon l'invention, un composé répulsif pour les invertébrés piqueurs hématophages est un composé présentant des propriétés répulsives pour les invertébrés piqueurs hématophages, c'est-à-dire un composé qui éloigne et donc repousse les invertébrés piqueurs hématophages.

Dans le cadre de la présente invention, on entend par « groupe alkyle » un groupe aliphatique hydrocarboné, saturé, linéaire ou ramifié comprenant, sauf mention contraire, de 2 à 20 atomes de carbone. A titre d'exemples, on peut citer les groupes éthyle, n-propyle, isopropyle, butyle, isobutyle, tertbutyle ou pentyle, ou encore nonyle ou décyle.

Selon un mode de réalisation, dans la formule (I-1) susmentionnée, R représente un groupe alkyle, linéaire ou ramifié, comprenant de 4 à 20 atomes de carbone.

Selon un mode de réalisation, dans la formule (I-1) susmentionnée, R représente un groupe alkyle, linéaire ou ramifié, comprenant de 4 à 16, notamment de 4 à 10, atomes de carbone.

Comme indiqué plus haut, lorsque R est un groupe linéaire dans la formule (I-1) telle que définie ci-dessus, le composé correspondant ne présente pas de carbone asymétrique et est donc un composé achiral.

Lorsque R est un groupe ramifié dans la formule (I-1) telle que définie ci-dessus, le composé correspondant est alors sous forme d'un mélange racémique, d'un énantiomère (*R*) ou d'un énantiomère (*S*).

La présente invention concerne également l'utilisation d'un composé de formule (II) suivante :

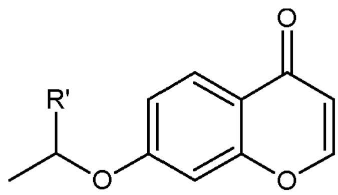
(II)

R' représentant un groupe alkyle linéaire comprenant de 2 à 18 atomes de carbone, ledit composé étant sous forme d'un mélange racémique, d'un énantiomère (*R*) ou d'un énantiomère (*S*), comme attractif ou répulsif pour les invertébrés piqueurs hématophages.

Selon un mode de réalisation non compris dans l'invention revendiquée, l'utilisation susmentionnée d'un composé de formule (III) peut être envisagée:

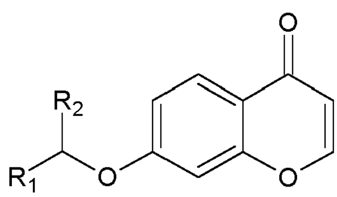
(III)

dans laquelle :

- soit $R_1$ est H ou un groupe méthyle et $R_2$ représente un groupe alkyle comprenant de 3 à 18 atomes de carbone,
- soit $R_2$ est H ou un groupe méthyle et $R_1$ représente un groupe alkyle comprenant de 3 à 18 atomes de carbone,

comme attractif pour les invertébrés piqueurs hématophages,
ledit composé étant sous forme d'un mélange racémique, d'un énantiomère (*R*) ou d'un énantiomère (*S*).

La présente invention concerne également l'utilisation susmentionnée d'un composé de formule (II) :

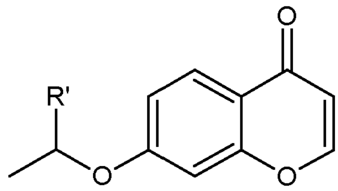

(II)

dans laquelle R' représente un groupe alkyle comprenant de 3 à 18 atomes de carbone, comme attractif pour les invertébrés piqueurs hématophages,
ledit composé étant sous forme d'un mélange racémique, d'un énantiomère (R) ou d'un énantiomère (*S*).

Selon un mode de réalisation non compris dans l'invention revendiquée, l'utilisation susmentionnée d'un composé de formule (II-1) peut être envisagée:

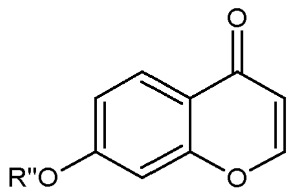

(II-1)

dans laquelle R" représente un groupe alkyle linéaire comprenant de 4 à 20 atomes de carbone, comme attractif pour les invertébrés piqueurs hématophages,
ledit composé étant sous forme d'un mélange racémique, d'un énantiomère (*R*) ou d'un énantiomère (S).

La présente invention concerne également l'utilisation d'un composé de formule (II) telle que définie ci-dessus, dans laquelle R' représente un groupe alkyle comprenant 2 atomes de carbone, comme répulsif pour les invertébrés piqueurs hématophages, ledit composé étant sous forme d'un mélange racémique, d'un énantiomère (*R*) ou d'un énantiomère (*S*).

Selon un mode de réalisation, le composé de formule (II) pour l'utilisation susmentionnée est sous forme de l'énantiomère (*R*) ou d'un mélange racémique.

Selon un mode de réalisation, le composé de formule (II) pour l'utilisation susmentionnée est sous forme de l'énantiomère (*S*), et le composé est utilisé dans une composition dans laquelle la teneur dudit composé est supérieure ou égale à 20% en poids par rapport au poids total de ladite composition.

La présente invention concerne également l'utilisation susmentionnée, d'un composé de formule (II) comme attractif pour les invertébrés piqueurs hématophages, dans laquelle R' représente un groupe alkyle comprenant 2 atomes de carbone, ledit composé étant sous forme de l'énantiomère (*S*), et dans laquelle le composé est utilisé dans une composition dans laquelle la teneur dudit composé est inférieure ou égale à 20% en poids par rapport au poids total de ladite composition.

A titre de composés de formule (I-1) utilisés selon l'invention, les composés suivants peuvent notamment être mentionnés : 7-*sec*-butoxychromone ; 7-*sec*-pentoxychromone ; 7-sec-nonyloxychromone ; 7-R-(-)-*sec*-butoxychromone ; 7-S-(+)-sec-butoxychromone ; R-(-)-sec-pentoxychromone ; 7-n-décyloxychromone et 7-(2'-ethyl)hexyloxychromone.

La présente invention concerne également des compositions comprenant les composés susmentionnés de formule (I-1), (II), (III) ou (II-1), présentant des propriétés attractives ou répulsives.

La présente invention concerne également une composition attractive ou répulsive pour des invertébrés piqueurs hématophages, comprenant au moins un composé de formule (I-1) telle que définie ci-dessus, ledit composé étant sous forme d'un mélange racémique, d'un énantiomère (*R*) ou d'un énantiomère (*S*),

ladite composition comprenant éventuellement en outre un composé additionnel attractif ou répulsif pour un invertébré piqueur hématophage.

La présente invention concerne également une composition attractive pour des invertébrés piqueurs hématophages telle que définie ci-dessus, comprenant :

- au moins un composé de formule (I-1) telle que définie ci-dessus, dans laquelle R représente un groupe alkyle comprenant de 2 à 16 atomes de carbone, de préférence de 5 à 10 atomes de carbone, ou
- au moins un composé de formule (I-1) telle que définie ci-dessus, dans laquelle R représente un groupe alkyle comprenant 4 atomes de carbone, ledit composé étant sous forme de l'énantiomère (*S*), et dans laquelle le composé sous forme de l'énantiomère (*S*) est utilisé en une teneur inférieure ou égale à 20% de ladite composition.

La présente invention concerne également une composition attractive pour des invertébrés piqueurs hématophages telle que définie ci-dessus, comprenant :

- au moins un composé de formule (II) telle que définie ci-dessus, dans laquelle R' représente un groupe alkyle comprenant de 3 à 18 atomes de carbone, de préférence de 3 à 10 atomes de carbone, ou
- au moins un composé de formule (II) telle que définie ci-dessus, dans laquelle R' représente un groupe alkyle comprenant 2 atomes de carbone, ledit composé étant sous forme de l'énantiomère (*S*), et dans laquelle le composé sous forme de l'énantiomère (*S*) est utilisé en une teneur inférieure ou égale à 20% de ladite composition.

La présente invention concerne également une composition répulsive pour des invertébrés piqueurs hématophages telle que définie ci-dessus, comprenant :

- au moins un composé de formule (I-1) dans laquelle R représente un groupe alkyle comprenant 4 atomes de carbone, le composé de formule (I-1) étant sous forme de l'énantiomère (*R*) ou d'un mélange racémique, ou d'un mélange des énantiomères (*R*) et (*S*) en des teneurs massiques différentes de 50/50% ; ou
- au moins un composé de formule (I-1) dans laquelle R représente un groupe alkyle comprenant 4 atomes de carbone, ledit composé étant sous forme de l'énantiomère (*S*) et dans laquelle ledit composé sous forme de l'énantiomère (*S*) est utilisé en une teneur supérieure ou égale à 1% en poids par rapport au poids de ladite composition.

La présente invention concerne également une composition répulsive pour des invertébrés piqueurs hématophages telle que définie ci-dessus, comprenant :

- au moins un composé de formule (II) dans laquelle R' représente un groupe alkyle comprenant 2 atomes de carbone, le composé de formule (I) étant sous forme de l'énantiomère (*R*) ou d'un mélange racémique, ou d'un mélange des énantiomères (*R*) et (*S*) en des teneurs massiques différentes de 50/50% ; ou
- au moins un composé de formule (II) dans laquelle R' représente un groupe alkyle comprenant 2 atomes de carbone, ledit composé étant sous forme de l'énantiomère (*S*) et dans laquelle ledit composé sous forme de l'énantiomère (*S*) est utilisé en une teneur supérieure ou égale à 1% en poids par rapport au poids de ladite composition.

Selon un mode de réalisation, les compositions répulsives selon l'invention sont sous forme de spray ou de crème, de support, par exemple de support textile ou de filets, ou de cartouches pour diffuseurs.

Selon un mode de réalisation, les compositions de l'invention sont des compositions attractives ou répulsives pour des moustiques choisis dans le groupe constitué des espèces : *Aedes albopictus, Aedes aegypti, Anopheles gambiae s.l*, *Anopheles mascarensis, Anopheles funestus*, *Anopheles stephensi*, *Culex pipiens quinquefasciatus* et *Culex pipiens pipiens.*

Les compositions de l'invention peuvent en outre comprendre un composé additionnel attractif ou répulsif pour un invertébré piqueur hématophage. Cette combinaison permet d'améliorer encore les propriétés attractives ou répulsives de la composition de l'invention.

Par exemple, une composition attractive de l'invention peut comprendre la molécule attractive 4-hydroxycoumarine, en association avec un composé de formule (I-1) ou (II), (II-1) ou (III) telles que définies ci-dessus. Une telle combinaison est synergique et permet d'améliorer les propriétés attractives du composé (I-1), (II), (II-1) ou (III) par rapport à une composition comprenant uniquement un composé (I-1), (II), (II-1) ou (III).

La présente invention concerne également un kit pour le piégeage d'un invertébré piqueur hématophage, notamment de moustique, et en particulier *Aedes albopictus,* comprenant :

- au moins une composition attractive comprenant au moins un composé de formule (I-1) suivante :

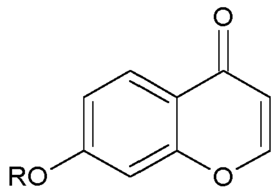

(I-1)

R représentant un groupe alkyle, linéaire ou ramifié, comprenant de 2 à 20 atomes de carbone,

ledit composé étant achiral lorsque R est un groupe linéaire ou sous forme d'un mélange racémique, d'un énantiomère (*R*) ou d'un énantiomère (*S*) lorsque R est un groupe ramifié,

- ladite composition comprenant éventuellement en outre un composé additionnel attractif pour un invertébré piqueur hématophage , et
- au moins un piège pour invertébré piqueur hématophage, notamment de moustique, et en particulier *Aedes albopictus.*

La présente invention concerne également un kit pour le piégeage d'un invertébré piqueur hématophage tel que défini ci-dessus, dans lequel la composition attractive comprend :

- au moins un composé de formule (I-1), dans laquelle R représente un groupe alkyle comprenant de 2 à 16 atomes de carbone, ou
- au moins un composé de formule (I-1), dans laquelle R représente un groupe alkyle comprenant 4 atomes de carbone, ledit composé étant sous forme de l'énantiomère (S), et dans laquelle le composé sous forme de l'énantiomère (S) est utilisé en une teneur inférieure ou égale à 20% de ladite composition.

Les pièges utilisés selon l'invention sont ceux bien connus de l'homme du métier.
A titre de piège, on peut notamment citer le piège sentinelle BG (Biogent®) ou encore le piège lumineux CDC pour capturer *Aedes* sp. et *Anopheles* sp., respectivement.
La présente invention concerne également l'utilisation du kit susmentionné, pour le piégeage d'invertébrés piqueurs hématophages, notamment de moustiques, en particulier *Aedes albopictus* et *Anopheles sp.*
La présente invention concerne également un support répulsif pour les invertébrés piqueurs hématophages, notamment pour les moustiques, et en particulier *Aedes albopictus,* ledit support étant imprégné d'au moins une composition répulsive comprenant:

- au moins un composé de formule (I-1) suivante :

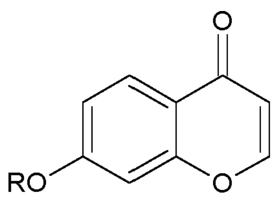

(I-1)

dans laquelle R représente un groupe alkyle comprenant 4 atomes de carbone, le composé de formule (I) étant sous forme de l'énantiomère (*R*) ou d'un mélange racémique, ou d'un mélange des énantiomères (*R*) et (*S*) en des teneurs massiques différentes de 50/50% ; ou
- au moins un composé de formule (I) dans laquelle R représente un groupe alkyle comprenant 4 atomes de carbone, ledit composé étant sous forme de l'énantiomère (*S*) et dans laquelle ledit composé sous forme de l'énantiomère (*S*) est utilisé en une teneur supérieure ou égale à 20% en poids par rapport au poids de ladite composition, ladite composition comprenant éventuellement en outre un composé additionnel répulsif pour un invertébré piqueur hématophage, ledit support étant notamment choisi dans le groupe constitué des textiles, en particulier des moustiquaires, des vêtements pour randonnées, des bracelets, colliers ou cartouches pour diffuseurs.

Un tel support répulsif est préparé en imprégnant un support tel que défini ci-dessus avec une composition répulsive selon l'invention telle que définie ci-dessus. Cette étape d'imprégnation peut être effectuée par exemple par pulvérisation ou trempage.

**FIGURES**

La Figure 1 représente l'effet synergique entre les composés 4-hydroxycoumarine et 7-nonyloxychromone vis-à-vis de *Aedes albopictus.* Elle représente l'IK (en %) en fonction de la quantité déposée (en mg). La courbe avec les losanges correspond à la 4-hydroxycoumarine, la courbe avec les carrés correspond au 7-nonyloxychromone et la courbe avec les triangles correspond à la synergie.

La Figure 2 représente l'IK (en %) en fonction de la quantité déposée (en mg) pour la 7-n-décyloxychromone vis-à-vis *d'Aedes albopictus.*

La Figure 3 représente l'IK (en %) en fonction de la quantité déposée (en mg) pour la 7-(2'-ethyl)hexyloxychromone vis-à-vis d'*Aedes albopictus.*

**EXEMPLES**

**MATÉRIELS ET MÉTHODES**

**SYNTHÈSE DES DÉRIVÉS DE CHROMONE**

Le 7-hydroxy-4-chromone est utilisé comme le produit de départ afin d'obtenir les dérivés éthers. La synthèse est effectuée en fonction de l'effet de la structure de la molécule sur le comportement des moustiques.

***1. Synthèse par catalyse par transfert de phase***

La réaction avec un catalyseur de transfert de phase est utilisée pour la synthèse des produits non chiraux comme indiqué ci-dessous :

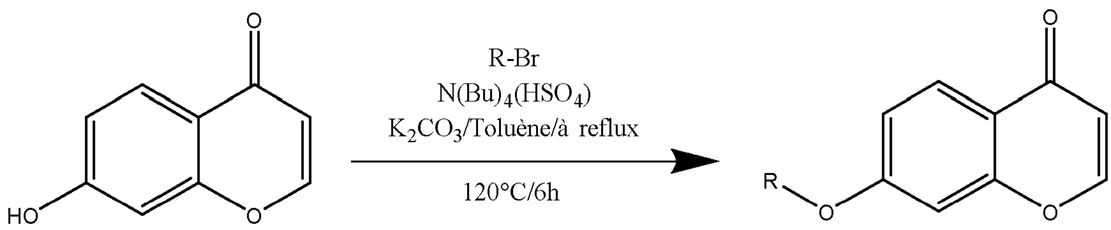


**Exemple 1 : Synthèse de la 7-*sec*-butoxychromone racémique** (1CPT)

Dans un réacteur tricol de 250 ml monté à reflux équipé d'un agitateur magnétique et d'un thermomètre, on ajoute 100 ml de toluène et 8,4 g de $K_2CO_3$ (61 mmol), puis 2 g de 7-hydroxychromone (12 mmol) et 10,14 g de 2-bromobutane (82 mmol) sont ajoutés dans le mélange. Pour le catalyseur de transfert de phase, 0,1 g de tétrabutyl ammonium hydrogénosulfate est ajouté. Le mélange est agité au reflux pendant 6 h à 120°C et la phase organique est récupérée par décantation, lavée à l'eau distillée, séchée avec $Na_2SO_4$ puis évaporée. Le mélange huileux résultant est purifié par chromatographie sur colonne avec gel de silice (éluant acétate d'éthyle 2 hexane 8). Le produit pur est obtenu sous forme d'une huile jaune avec un rendement égal à 73%.

**Exemple 2 : Synthèse de la 7-*sec*-pentoxychromone racémique** (2CPT)

Dans un réacteur tricol de 250 ml monté à reflux équipé d'un agitateur magnétique et d'un thermomètre, on ajoute 100 ml de toluène et 4,2 g de $K_2CO_3$ (30,6 mmol), puis 1 g de 7-hydroxychromone (6,15 mmol) et 5 g de 2-bromopentane (41,2 mmol) sont ajoutés dans le mélange. Pour le catalyseur de transfert de phase, 0,05 g de tétrabutyl ammonium hydrogénosulfate est ajouté. Le mélange est agité au reflux pendant 6 h à 120°C et la phase organique est récupérée par décantation, lavée à l'eau distillée, séchée avec $Na_2SO_4$ puis évaporée. Le mélange huileux est purifié par chromatographie sur colonne avec gel de silice (éluant acétate d'éthyle 2 hexane 8). Le produit pur est obtenu sous forme d'une huile jaune avec un rendement égal à 33,1%.

**Exemple 3 : Synthèse de la 7-sec-nonyloxychromone racémique** (3CPT)

Dans un réacteur tricol de 250 ml monté à reflux équipé d'un agitateur magnétique et d'un thermomètre, on ajoute 100 ml de toluène et 2,13 g de $K_2CO_3$ (15,3 mmol), puis 0,5 g de 7-hydroxychromone (3,1 mmol) et 3 g de 2-bromononane (20,6 mmol) sont ajoutés dans le mélange. Pour le catalyseur de transfert de phase, 0,025 g de tétrabutyl ammonium hydrogénosulfate est ajouté. Le mélange est agité au reflux pendant 6 h à 120°C et la phase organique est récupérée par décantation, lavée à l'eau distillée, séchée avec $Na_2SO_4$ puis évaporée. Le mélange huileux est purifié par chromatographie sur colonne avec gel de silice (éluant acétate d'éthyle 2 hexane 8). Le produit pur est obtenu sous forme d'une huile jaune avec un rendement égal à 35.2%.

**Exemple 4 : Synthèse de la 7-isopropyloxychromone** (4CPT)

Dans un tube scellé de 15 ml équipé d'un agitateur magnétique et d'un thermomètre, on ajoute 7 ml de toluène et 0.8 g de $K_2CO_3$ (5,8 mmol), 0,25 g de 7-hydroxychromone (1,54 mmol), puis 2 g de 2-bromopropane (16,26 mmol) sont ajoutés dans le mélange. Comme catalyseur de transfert de phase, 0,4 g de tétrabutyl ammonium hydrogénosulfate est ajouté. Le mélange est agité pendant 24 heures à 90°C et la phase organique est récupérée par décantation, lavée à l'eau distillée, séchée avec $Na_2SO_4$ puis évaporée. Le mélange huileux est purifié par chromatographie sur colonne avec gel de silice (éluant acétate d'éthyle 2 hexane 8). Le produit pur est obtenu sous forme de solides blancs avec un rendement égal à 29,4%.

***2. Synthèse par la réaction de Mitsunobu***

La réaction de Mitsunobu est utilisée pour transformer sélectivement en une seule étape un alcool en différents groupements fonctionnels comme l'éther. Cette réaction permet aussi une énantio-inversion des alcools chiraux, comme indiqué ci-dessous :

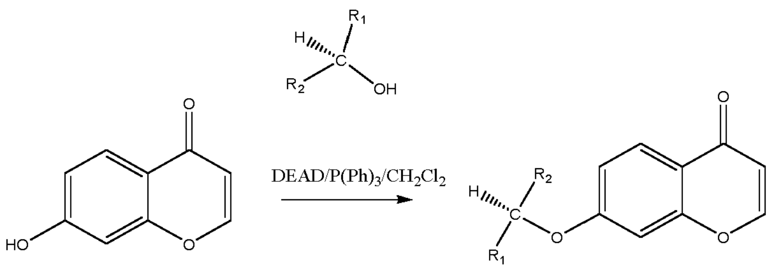


**Exemple 5 : Synthèse de la *R*-(-)-*sec*-butoxychromone** (5M)

Dans un réacteur de 50 ml équipé d'un thermomètre, d'un agitateur magnétique et d'une légère surpression d'azote, on ajoute successivement 15 ml de dichlorométhane, 1 g de 7-hydroxychromone (6,2 mmol), puis 1,2 ml de S-butan-2-ol (12,4 mmol), 3,2 g de triphénylphosphine (12,4 mmol). Le mélange est ensuite agité quelques minutes puis on ajoute goutte à goutte 2,2 ml de solution de DEAD (diéthyl azodicarboxylate) (24 mmol). La réaction est effectuée à température ambiante pendant 15 à 24 heures. Puis, la solution est évaporée et le mélange est purifié par chromatographie sur colonne avec gel de silice (éluant acétate d'éthyle 2 hexane 8). Le produit pur est obtenu sous forme d'une huile jaune pâle avec un rendement égal à 52,6%.

**Exemple 6 : Synthèse de la *S*-(+)-*sec*-butoxychromone** (6M)

Dans un réacteur de 50 ml équipé d'un thermomètre, d'un agitateur magnétique et d'une légère surpression d'azote, on ajoute successivement 30 ml de dichlorométhane, 2 g de 7-hydroxychromone (12,4 mmol), puis, 2.4 ml de R-butan-2-ol (24,6 mmol), 6,4 g de triphénylphosphine (24,6 mmol). Le mélange est ensuite agité quelques minutes puis on ajoute goutte à goutte 4,2 ml de solution de DEAD (24 mmol). La réaction est effectuée à température ambiante pendant 15 à 24 heures. Puis, la solution est évaporée et le mélange est purifié par chromatographie sur colonne avec gel de silice (éluant acétate d'éthyle 2 hexane 8). Le produit pur est obtenu sous forme d'une huile rose pâle avec un rendement égal à 53%.

**Exemple 7 : Synthèse de la (*R*) 7-*sec*-pentoxychromone** (7M)

Dans un réacteur de 50 ml équipé d'un thermomètre, d'un agitateur magnétique et d'une légère surpression d'azote, on ajoute successivement 30 ml de dichlorométhane, 1 g de 7-hydroxychromone (6,2 mmol), puis 1,4 ml de S-pentan-2-ol (12,3 mmol), 3,23 g de triphénylphosphine (12,3 mmol). Le mélange est ensuite agité quelques minutes puis on ajoute goutte à goutte 2,1 ml de solution de DEAD (12,3 mmol). La réaction est effectuée à température ambiante pendant 15 à 24 heures. Puis, la solution est évaporée et le mélange est purifié par chromatographie sur colonne avec gel de silice (éluant acétate d'éthyle 2 hexane 8). Le produit pur est obtenu sous forme d'une huile rose pâle avec un rendement égal à 63,3%.

**Exemple 8 : Synthèse de la (*S*) 7-sec-pentoxychromone** (8M)

Dans un réacteur de 50 ml équipé d'un thermomètre, d'un agitateur magnétique et d'une légère surpression d'azote, on ajoute successivement 30 ml de dichlorométhane, 1 g de 7-hydroxychromone (6,2 mmol), puis 1,4 ml de R-pentan-2-ol (12,3 mmol), 3,23 g de triphénylphosphine (12,3 mmol). Le mélange est ensuite agité quelques minutes puis on ajoute goutte à goutte 2,1 ml de solution de DEAD (12,3 mmol). La réaction est effectuée à température ambiante pendant 15 à 24 heures. Puis, la solution est évaporée et le mélange est purifié par chromatographie sur colonne avec gel de silice (éluant acétate d'éthyle 2 hexane 8). Le produit pur est obtenu sous forme d'une huile jaune pâle avec un rendement égal à 42,4%.

**Exemple 4bis : Synthèse de la 7-*n*-décyloxychromone** (5CPT)

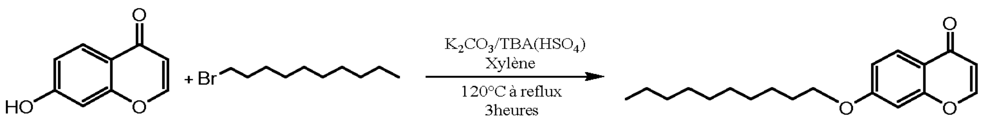


Dans un réacteur tricol de 50 ml monté à reflux équipé d'un agitateur magnétique et d'un thermomètre, on ajoute 10 ml de toluène et 0,691 g de $K_2CO_3$ (5 mmol), puis 0,2 g de 7-hydroxychromone (1 mmol) et 0.660 g de 1-bromodecane (3 mmol) sont ajoutés dans le mélange. Pour le catalyseur de transfert de phase, 0,05 g de tétrabutyl ammonium hydrogénosulfate est ajouté. Le mélange est agité au reflux pendant 3 h à 120°C et la phase organique est récupérée par décantation, lavée à l'eau distillée, séchée avec $Na_2SO_4$ puis évaporée. Le mélange huileux est purifié par extraction par partage liquide-liquide (hexane et eau distillée). Le produit pur est obtenu sous forme d'un solide blanc (Pf= 45°C) avec un rendement égal à 66%.

**Exemple 4ter : Synthèse de la 7-(2'-ethyl)hexyloxychromone**

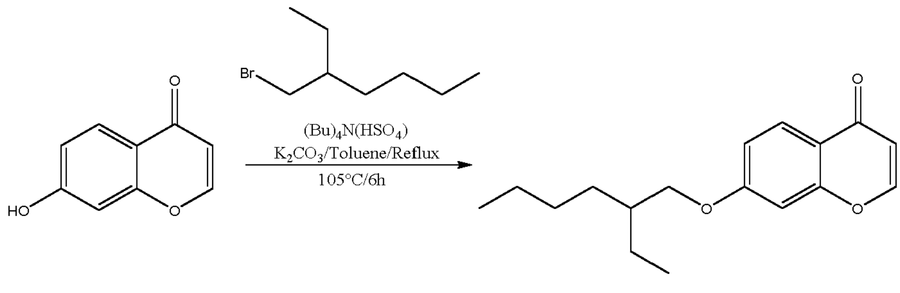


Dans un réacteur tricol de 50 ml monté à reflux équipé d'un agitateur magnétique et d'un thermomètre, on ajoute 10 ml de toluène et 0,691 g de $K_2CO_3$ (5 mmol), puis 0,2 g de 7-hydroxychromone (1.2 mmol) et 0,580 g de 1-bromo-2-ethylhexane (3 mmol) sont ajoutés dans le mélange. Pour le catalyseur de transfert de phase, 0,02 g de tétrabutyl ammonium hydrogénosulfate est ajouté. Le mélange est agité au reflux pendant 6 h à 120°C et la phase organique est récupérée par décantation, lavée à l'eau distillée, séchée avec $Na_2SO_4$ puis évaporée. Le mélange brut est purifié sur chromatographie sur colonne avec gel de silice (éluant hexane/acétate d'éthyle 98/2). Le produit pur est obtenu sous forme d'huile jaune avec un rendement égal à 65%.

## ANALYSE CHIMIQUE

Les spectres RMN $^1H$ et $^{13}C$ des produits synthétisés sont obtenus avec le spectromètre BRUKER.

Un polarimètre ATAGO POLAX-D est utilisé pour déterminer les pouvoirs rotatoires des produits chiraux avec un tube observateur de 2 dm. Les produits sont dilués dans l'éthanol à 0,2%.

## EVALUATION DE L'EFFET RÉPULSIF ET/OU ATTRACTANT DES ÉTHERS DE CHROMONE DANS UN OLFACTOMÈTRE EN TUNNEL

L'olfactomètre en tunnel est un parallélépipède de verre de 1m de long et de 5×5 cm de côté. Le tube est équipé de 3 ouvertures : deux de chaque côté permettant l'introduction du papier imprégné du produit à tester et de l'autre le papier contenant un témoin. L'ouverture du milieu permet l'introduction des moustiques. Les deux extrémités du dispositif sont fermées par des couvercles.

L'olfactomètre est divisé en trois compartiments ou zones :

- une zone neutre qui se trouve au milieu ;
- une zone test ou traitée qui se trouve du côté de dépôt de produit ; et
- une zone contrôle qui se trouve du côté de dépôt de témoin.

Pour chaque test, 15 femelles *Aedes albopictus* (âgées de 5 à 12 jours) sont introduites dans la zone neutre de l'olfactomètre en tunnel. Les moustiques sont maintenus 10 minutes pour un temps d'adaptation. Les papiers imprégnés et séchés d'une solution de produit dans l'éthanol et d'éthanol pur comme référence sont introduits, puis les barrières sont ouvertes pour permettre aux moustiques de circuler librement à l'intérieur du dispositif. Trois répétitions de test ont été effectuées pour chaque produit. Une répétition dure 20 minutes et les résultats observés ont été enregistrés toutes les 5 minutes.

Les valeurs relevées sont l'indice d'activité et l'indice de répulsion. L'indice d'activité (AI) décrit le pourcentage de moustiques se trouvant dans la zone témoin (T) et la zone traitée (P) par rapport au nombre total de moustiques testés. Cette valeur doit être supérieure à 30% pour que l'essai soit considéré comme significatif.

L'indice de répulsion (RI) représente le pourcentage de la différence du nombre de moustiques dans la partie témoin et la partie traitée divisée par la somme de ces deux valeurs. Une valeur négative du paramètre (RI) indique une activité attractante (Kairomone indice, KI) du produit testé.

$$\text{IA} = \left(\frac{T+P}{15}\right) * 100 \qquad \text{IR} = \left(\frac{T-P}{T+P}\right) * 100$$

IA : indice d'activité
IR : indice de répulsion
T : nombre de moustiques dans la zone témoin
P : nombre de moustiques dans la zone traitée

## RÉSULTATS

## SYNTHÈSE DES DÉRIVÉS DE CHROMONE

### *1. Synthèse par catalyse par transfert de phase*

Cinq produits ont été synthétisés en fonction de la longueur de la chaîne alkyle : le 7-sec-butoxychromone, la 7-sec-pentoxychromone, la 7-sec-nonyloxychromone, la 7-isopropyloxychromone et le 7-décyloxychromone. Les résultats obtenus sont illustrés dans le tableau 1.

**Tableau 1.** Résultats obtenus de la synthèse des éthers de chromone non chiraux par la réaction catalyse par transfert de phase

| Entrée | **Halogénoalcane** | **Produit obtenu** | **Rendement pur** | **Aspect** |
|---|---|---|---|---|
| **1 CPT** | 2-bromobutane | 7-sec-butoxychromone | 73% | Huile jaune |
| **2 CPT** | 2-bromopentane | 7-sec-pentoxychromone | 33,1% | Huile jaune |
| **3 CPT** | 2-bromononane | 7-sec-nonyloxychromone | 35,2% | Huile jaune |

(suite)

| Entrée | Halogénoalcane | Produit obtenu | Rendement pur | Aspect |
|---|---|---|---|---|
| **4 CPT** | 2-bromopropane | 7-isopropyloxychromone | 29,4% | Solide blanc |
| **5 CPT** | 1-bromodécane | 7-n-décyloxychromone | 66% | Solide jaune |

***2. Synthèse par la réaction de Mitsunobu***

Quatre énantiomères ont été synthétisés : la *R*-(-)-*sec*-butoxychromone, le *S*-(+)-*sec*-butoxychromone, la *R*-(-)-*sec*-pentoxychromone et la *S*-(+)-*sec*-pentoxychromone. Le tableau 2 montre les résultats obtenus durant les réactions de synthèse.

**Tableau 2.** Résultats obtenus de la synthèse des produits chiraux par la réaction de Mitsunobu

| Entrée | Alcool chiral | Produit obtenu | Pouvoir rotatoire (0,2% dans EtOH) | Rendement pur | Aspect |
|---|---|---|---|---|---|
| **5 M** | (*S*) butan-2-ol | *R*-(-)-*sec*-butoxychromone | -35 | 52,6% | Huile jaune pâle |
| **6 M** | (*R*) butan-2-ol | *S*-(+)-*sec*-butoxychromone | +35 | 53% | Huile rose pâle |
| **7 M** | (*R*) pentan-2-ol | *R*-(-)-*sec*-pentoxychromone | -22,5 | 63,3% | Huile rose pâle |
| **8 M** | (*S*) pentan-2-ol | *S*-(+)-*sec*-pentoxychromone | +22.5 | 42.4% | Huile rose pâle |

**ANALYSE CHIMIQUE DES PRODUITS PAR SPECTROMÉTRIE MAGNÉTIQUE NUCLÉAIRE (RMN)**

**7-*sec*-butoxychromone racémique** (1CPT)

RMN $^1H$ (300 MHz) dans $CDCl_3$ : 8.11 (1H, d, $H_5$ ), 7.77 (1H, d, $H_2$), 6.96 (1H, dd, $H_6$), 6.82 (1H, s, $H_8$ ), 6.26 (1H, d, $H_3$), 4.41 (1H, m, $H_{11}$), 1.77 (2H, m, $H_{13}$ ), 1.36 (3H, d, $H_{12}$ ), 1 (3H, t, $H_{14}$ )
RMN$^{13}C$ dans $CDCl_3$ : 177.05, 162.90, 158.31, 154.83, 127.18, 118.40, 115.49, 112.83, 101.82, 75.82, 28.99, 9.68

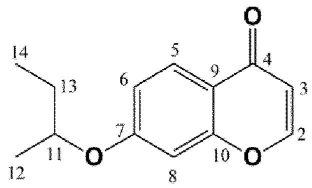

Rendement = 73% (Huile jaune pâle)

**7-*sec*-pentoxychromone** (2CPT)

RMN $^1H$ (300 MHz) dans $CDCl_3$ : 8.13 (1H, d, $H_5$ ), 7.75 (1H, d, $H_2$ ), 6.9 (1H, dd, $H_6$), 6.8 (1H, s, $H_8$ ), 6.28 (1H, d, $H_3$ ), 4.5 (1H, m, $H_{11}$ ), 1.75 (2H, m, $H_{13}$ ), 1.6 (3H, d, $H_{12}$ ), 1.38 (2H, m, $H_{14}$ ), 0.90 (3H, t, $H_{15}$ )
RMN $^{13}C$ dans $CDCl_3$ : 177.03, 162.90, 158.31, 154.79, 127.19, 118.40, 115.45, 112.84, 101.75, 74.42, 38.31, 19.44, 18.64, 13.95

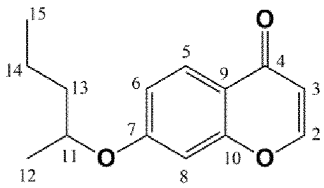

Rendement = 33.1% (Huile jaune pâle)

**7-*sec*-nonyloxychromone** (3CPT)

RMN $^1H$ (300 MHz) dans $CDCl_3$ : : 8.09 (1H, d, $H_5$ ), 7.75 (1H, d, $H_2$ ), 6.93 (1H, dd, $H_6$), 6.8 (1H, s, $H_8$ ), 6.28 (1H, d, $H_3$ ), 4.46 (1H, m, $H_{11}$ ), 1.76 (2H, m, $H_{13}$ ), 1.62 (3H, d, $H_{12}$ ), 1.41 (2H, m, $H_{18}$ ), 1.34 (2H, t, $H_{17}$ ), 1.34 (2H, t, $H_{16}$ ), 1.28 (2H, t, $H_{15}$ ), 1.28 (2H, t, $H_{14}$ ), 0.87 (3H, t, $H_{19}$)
RMN$^{13}C$ dans $CDCl_3$ : 177, 162.88, 158.30, 154.77, 127.18, 118.40, 115.44, 112.84, 101.77, 74.69, 36.18, 31.75, 29.46, 29.18, 25.40, 22.61, 19.45, 14.05

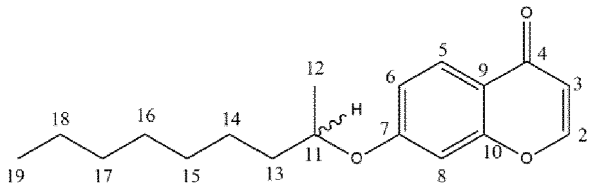

Rendement = 35.2% (Huile jaune pâle)

**7-isopropyloxychromone** (4CPT)

RMN $^1H$ (300 MHz) dans $CDCl_3$ :8.09 (1H, d, $H_5$ ), 7.76 (1H, d, $H_2$ ), 6.93 (1H, dd, $H_6$), 6.8 (1H, s, $H_8$ ), 6.26 (1H, d, $H_3$ ), 4.64 (1H, m, $H_{11}$ ), 1.35 (3H, d, $H_{12}$ ), 1.35 (3H, d, $H_{13}$ ),
RMN$^{13}C$ dans $CDCl_3$ : 176.98, 162.52, 158.27, 154.77, 127.15, 118.43, 115.43, 112.84, 101.81, 70.73, 21.77

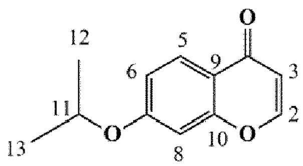

Rendement = 29.4% (Solide blanc)

**7-décyloxychromone**

RMN $^1H$ (300 MHz) dans $CDCl_3$ : 8.02 (1H, d, $H_5$), 7.68 (1H, d, $H_2$), 6.89 (1H, dd, $H_6$), 6.75 (1H, d, $H_8$), 6.20 (1H, d, $H_3$), 3.87-3.85 (2H, d, $H_{11}$), 1.72 (1H, m, $H_{12}$), 1.49 (2H, m, $H_{13}$), 1.39(2H, m, $H_{17}$), 1.26 (2H,m, $H_{16}$), 1.19 (2H, m, $H_{15}$), 0.90(3H, m, $H_{14}$), 0.85 (3H, m, $H_{18}$),
RMN $^{13}C$ dans $CDCl_3$ : 177.98 ($C_4$), 164.24 ($C_7$), 158.49 ($C_{10}$), 155.60 ($C_2$), 126.79 ($C_5$), 118.08 ($C_9$), 115.32 ($C_3$), 112.34 ($C_6$), 100.78 ($C_8$), 71.21 ($C_{11}$), 39.15 ($C_{12}$), 30.36 ($C_{15}$), 28.95 ($C_{16}$), 23.72 ($C_{13}$), 22.88 ($C_{17}$), 13.83 ($C_{18}$), 10.88 ($C_{14}$)

**7-(2'-ethyl)hexyloxychromone**

RMN $^1H$ (300 MHz) dans $CDCl_3$ : 8.03 (1H, d, $H_5$), 7.68 (1H, d, $H_2$), 6.84 (1H, dd, $H_6$), 6.75 (1H, d, $H_8$), 6.20 (1H, d, $H_3$), 3.87-3.85 (2H, d, $H_{11}$), 1.72 (1H, m, $H_{12}$),1.49 (2H, m, $H_{13}$), 1.39(2H, m, $H_{17}$), 1.26 (2H,m, $H_{16}$), 1.19 (2H, m, $H_{15}$), 0.90(3H, m, $H_{14}$), 0.85 (3H, m, $H_{18}$),
RMN $^{13}C$ dans $CDCl_3$ : 177.98 ($C_4$), 164.24 ($C_7$), 158.49 ($C_{10}$), 155.60 ($C_2$), 126.79 ($C_5$), 118.08 ($C_9$), 115.32 ($C_3$), 112.34 ($C_6$), 100.78 ($C_8$), 71.21 ($C_{11}$), 39.15 ($C_{12}$), 30.36 ($C_{15}$), 28.95 ($C_{16}$), 23.72 ($C_{13}$), 22.88 ($C_{17}$), 13.83 ($C_{18}$), 10.88 ($C_{14}$)

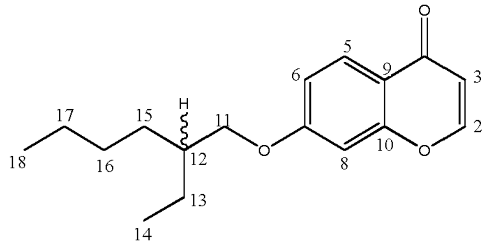

**ÉVALUATION DE L'EFFET RÉPULSIF ET/OU ATTRACTANT DES ÉTHERS DE CHROMONE DANS UN OLFACTOMÈTRE EN TUNNEL**

***Dérivés de chromones ayant des propriétés répulsives contre Aedes albopictus***

**Exemple 9 : Evaluation de la 7-sec-butoxychromone racémique** (1CPT)

Les résultats obtenus pour l'évaluation de l'activité répulsive ou attractante contre *Aedes albopictus* avec le composé 7-sec-butoxychromone racémique sont décrits dans le tableau 3 pour des doses de produits correspondant à 5, 10, 30 et 60 mg/ml.

**Tableau 3.** Comparaison par *t-test* des moyennes du nombre de moustiques dans les deux zones : contrôle et traitée. Indice d'activité et indice de répulsion pour le 7-*sec*-butoxychromone.

| Dose (mg/ml) | Produit (±SD) | Contrôle (±SD) | t | *p-value* | IA (%) | IR (%) |
|---|---|---|---|---|---|---|
| **5** | 3,3 ± 0,1 | 7,3 ± 0,4 | -12,8 | 0,0002 | 71,1 | 37,6 |
| **10** | 3,6 ± 0,4 | 7,2 ± 0,9 | -4,52 | 0,01 | 71,8 | 37,9 |
| **30** | 3,4 ± 0,9 | 8,3 ± 0,5 | -5,43 | 0,006 | 78 | 56,1 |
| **60** | 1,8 ± 0,2 | 11,8 ± 0,7 | -16,5 | <0,0001 | 90,6 | 72,9 |

**Exemple 10 : Evaluation de la *R*-(-)-sec-butoxychromone (5M)**

Les résultats obtenus pour l'évaluation de l'activité répulsive ou attractante contre *Aedes albopictus* avec le composé 7-*sec*-butoxychromone racémique sont décrits dans le tableau 3 pour des doses de produits correspondant à 5, 10, 30 et 60 mg/ml.

**Tableau 4.** Comparaison par *t-test* des moyennes du nombre de moustiques dans les deux zones : contrôle et traitée. Indice d'activité et indice de répulsion pour la *R*-(-)-*sec*-butoxychromone.

| Dose (mg/ml) | Traitée (±SD) | Contrôle (±SD) | t | *p-value* | IA (%) | IR (%) |
|---|---|---|---|---|---|---|
| **5** | 3,1 ± 0,1 | 9,5 ± 0,3 | -25,12 | < 0,0001 | 84 | 51,3 |
| **10** | 2,3 ± 0,7 | 11 ± 0,3 | -16,4 | < 0,0001 | 88,9 | 65,6 |
| **30** | 1,4 ± 0,1 | 12,4 ± 0,1 | -93,3 | < 0,0001 | 92,2 | 79,5 |
| **60** | 1,6 ± 0,2 | 12,2 ± 0,1 | -66,7 | < 0,0001 | 92 | 76,6 |

**Exemple 11 : Evaluation de la S-(+)-*sec*-butoxychromone (6M)**

Les résultats obtenus pour l'évaluation de l'activité répulsive ou attractante contre *Aedes albopictus* avec le composé 7-sec-butoxychromone racémique sont décrits dans le tableau 5 pour des doses de produits correspondant à 5, 10, 30 et 60 mg/ml.

**Tableau 5.** Comparaison par *t-test* des moyennes du nombre de moustiques dans les deux zones : contrôle et traitée. Indice d'activité et indice de répulsion pour la *S*-(+)-*sec*-butoxychromone.

| Dose (mg/ml) | Traitée (±SD) | Contrôle (±SD) | t | *p-value* | IA (%) | IR (%) |
|---|---|---|---|---|---|---|
| **5** | 9,0 ± 0,2 | 4,8 ± 0,5 | 10,4 | 0,0004 | 91,7 | -31,2 |
| **10** | 8,9 ± 1,1 | 4,2 ± 0,9 | 4,03 | 0,016 | 87,2 | -35,8 |
| **30** | 3,3 ± 0,2 | 9,3 ± 0,8 | -8,8 | 0,001 | 83,3 | 47,9 |
| **60** | 2,0 ± 0,0 | 11,4 ± 0,8 | -15,6 | < 0,0001 | 89,4 | 70,5 |

Les indices d'activités sont très élevés > 70% indiquant que les résultats sont fiables. Le racémique et le composé (*R*) sont répulsifs quelle que soit la dose. Le composé (S) est attractif à faible dose et répulsif à forte dose. Ce phénomène d'inversion de l'effet a déjà été observé sur certaines coumarines.

On note que le racémique a un effet correspondant sensiblement à la moyenne des effets des deux énantiomères.

**Exemple 12 : Comparaison de (R) 7-sec-butoxychromone avec la picaridine et le DEET**

On compare maintenant les propriétés répulsives du (*R*) 7-*sec*-butoxychromone avec ceux de répulsifs connus à doses équivalentes 10 et 30 mg/ml on observe que ce nouveau répulsif est autant et plus efficace que la Picaridine et le DEET qui sont considérés comme les produits les plus efficaces du marché.

**Tableau 6.** Comparaison des effets du (R) 7-sec-butoxychromone avec le DEET et la picaridine.

| | Indice de répulsion Dépôt de 5 mg | Indice de répulsion Dépôt de 10 mg | Indice de répulsion Dépôt de 30 mg |
|---|---|---|---|
| **(*R*) 7-*sec*-butoxychromone** | 51% | 65.6% | 79.5% |
| **DEET** | 55.5% | 60.4% | 59.1% |
| **Picaridine** | 42.6% | 49.6% | 59.7% |

***Dérivés de chromones ayant des propriétés attractives vis-à-vis de Aedes albopictus***

Il est tout aussi intéressant d'obtenir des produits attractifs des insectes nuisibles car cela permet la fabrication de pièges sélectifs et efficaces pour les insectes dangereux. Certaines chromones substituées ont montré des activités attractives.

Les synthèses de ces composés sont effectuées selon les mêmes procédés que pour les 7-sec-butoxychromones : par catalyse par transfert de phase pour le racémique et par réaction de Mitsunobu pour les énantiomères *R* et *S*.

**Exemple 13 : Evaluation de la 7-*sec*-pentoxychromone racémique ou (+/-) (2CPT)**

Les résultats obtenus pour l'évaluation de l'activité attractante vis-à-vis de *Aedes albopictus* avec le composé 7-*sec*-pentoxychromone racémique sont décrits dans le tableau 7 pour des doses de produits correspondant à 1, 5, 10 et 30 mg/ml.

**Tableau 7.** Comparaison par *t-test* des moyennes du nombre de moustiques dans les deux zones : contrôle et traitée. Indice d'activité et indice de répulsion pour la *7-sec*-pentoxychromone.

| Dose (mg/ml) | Traitée (±SD) | Contrôle (±SD) | t | *p-value* | IA (%) | IK (%) |
|---|---|---|---|---|---|---|
| **1** | 9,3 ± 0,7 | 3,5 ± 0,5 | 8,49 | 0,001 | 85 | -45,6 |
| **5** | 9,5 ± 0,2 | 2,5 ± 0,3 | 23,5 | < 0,0001 | 80,1 | -59,2 |
| **10** | 9,8 ± 1,1 | 3,4 ± 0,4 | 6,8 | 0,002 | 88,3 | -47,9 |
| **30** | 8,7 ± 0,4 | 4,8 ± 0,4 | 8,5 | 0,001 | 90 | -28,6 |

**Exemple 14 : Evaluation de la (R) 7-sec-pentoxychromone (6M)**

Les résultats obtenus pour l'évaluation de l'activité attractante vis-à-vis de *Aedes albopictus* avec le composé (*R*) 7-*sec*-pentoxychromone racémique sont décrits dans le tableau 8 pour des doses de produits correspondant à 1, 5, 10 et

30 mg/ml.

**Tableau 8.** Comparaison par *t-test* des moyennes du nombre de moustiques dans les deux zones : contrôle et traitée. Indice d'activité et indice de répulsion pour la *R*-(-)-*sec*-pentoxychromone

| Dose (mg/ml) | Traitée (±SD) | Contrôle (±SD) | t | *p-value* | IA (%) | IK (%) |
|---|---|---|---|---|---|---|
| **1** | 8,6 ± 0,2 | 5,3 ± 0,3 | 10 | 0,001 | 92,2 | -24,3 |
| **5** | 10,8 ± 0,6 | 2,2 ± 0,4 | 14,7 | 0,001 | 86,7 | -66,6 |
| **10** | 8,6 ± 0,1 | 3,6 ± 0,4 | 16 | < 0,0001 | 81,1 | -47,1 |
| **30** | 6,9 ± 0,3 | 6,6 ± 0,4 | 0,89 | 0,42 | 90 | -2,8 |

**Exemple 15 : Evaluation de la (*S*) 7-*sec*-pentoxychromone (7M)**

Les résultats obtenus pour l'évaluation de l'activité attractante vis-à-vis de *Aedes albopictus* avec le composé (*S*) 7-*sec*-pentoxychromone racémique sont décrits dans le tableau 9 pour des doses de produits correspondant à 1, 5, 10 et 30 mg/ml.

**Tableau 9.** Comparaison par *t-test* des moyennes du nombre de moustiques dans les deux zones : contrôle et traitée. Indice d'activité et indice de répulsion pour la *S*-(+)-*sec*-pentoxychromone.

| Dose (mg/ml) | Traitée (±SD) | Contrôle (±SD) | t | *p-value* | IA (%) | IK (%) |
|---|---|---|---|---|---|---|
| **1** | 10,3 ± 0,7 | 3,8 ± 0,3 | 10,3 | 0,0004 | 93,3 | -46,3 |
| **5** | 9,5 ± 0,7 | 3,1 ± 1,2 | 5,9 | 0,004 | 83,9 | -52,4 |
| **10** | 8,4 ± 0,1 | 5,8 ± 0,2 | 13,8 | 0,0001 | 95 | -18,2 |
| **30** | 6,5 ± 0,7 | 5,2 ± 0,3 | 2,15 | 0,09 | 77,8 | -10,7 |

Les deux énantiomères *R, S* et le racémique du 7-pentoxychromone sont attractifs vis-à-vis de *Aedes albopictus*. L'effet du racémique semble cumuler les effets des 2 énantiomères purs.

On observe une courbe en cloche comme observée avec des kairomones connues (acide isovalérique, 1 octen-3-ol) (Andrianjafy *et al.* 2017).

**Exemple 16 : Evaluation de la 7-*sec*-nonyloxychromone (3CPT)**

La 7-sec-nonyloxychromone est évaluée dans un olfactomètre en tunnel pour des doses de 2 à 30 mg/ml. Les résultats sont présentés dans le tableau 10.

**Tableau 10.** Comparaison par *t-test* des moyennes du nombre de moustiques dans les deux zones : contrôle et traitée. Indice d'activité et indice de répulsion pour la 7-*sec*-nonyloxychromone.

| Dose (mg/ml) | Traitée (±SD) | Contrôle (±SD) | t | *p-value* | IA (%) | IK (%) |
|---|---|---|---|---|---|---|
| **0.1** | 11,2 ± 0,6 | 2,5 ± 0,2 | 19,6 | < 0,0001 | 91,1 | -63,5 |
| **2** | 12,3 ± 0,0 | 1,4 ± 0,4 | 29,8 | < 0,0001 | 91,1 | -79,8 |
| **5** | 10,4 ± 0,1 | 2,2 ± 0,3 | 35 | < 0,0001 | 83,9 | -65,8 |
| **10** | 11,3 ± 0,0 | 2,5 ±0,5 | 15,1 | 0,0001 | 91,7 | -63,6 |
| **30** | 8.4 ± 0.2 | 4.8 ± 0.4 | 10.4 | 0.0004 | 88,3 | -27,1 |

Comme pour les 7-sec-pentoxychromone, on obtient une courbe en cloche avec une valeur maximale de IK proche de 80% pour une dose 2 mg/ml. L'activité attractive reste supérieure à 65% jusqu'à 10 mg/ml. Même à forte dose (30 mg/ml), la 7-nonyloxychromone reste attractif. Et en tenant compte que la tension de vapeur de ce produit devrait être faible du fait de sa masse moléculaire élevée, la durée d'efficacité sera longue.

**Exemple 17 : Evaluation des propriétés attractives de la 7-n-décyloxychromone vis-à-vis d'*Aedes albopictus***

Les résultats obtenus pour l'évaluation de l'activité attractante vis-à-vis d'*Aedes albopictus* avec le composé 7-n-décyloxychromone racémique sont présentés dans le tableau 11 pour la dose de produit correspondant à 10 mg/ml.

| Dose (mg/ml) | Traitée (±SD) | Contrôle (±SD) | IA (%) | IK (±SD) (%) |
|---|---|---|---|---|
| **10** | 8,8 ± 0,9 | 3,9±0,3 | 85 | -37,8±5,2 |

La 7-n-décyloxychromone a un effet attractif équivalent à celui de 7-*sec*-nonyloxychromone mais présente l'avantage d'être beaucoup moins coûteuse (halogénure primaire peu coûteux et bio-sourcé).

**Exemple 18 : Comparaison de 7-*sec*-pentoxychromone et de 7-sec-nonyloxychromone avec l'octen-3-ol**

On compare maintenant les propriétés attractives des racémiques 7-*sec*-pentoxychromone et 7-sec-nonyloxychromone avec l'octen-3-ol.

**Tableau 12.** Comparaison de 7-*sec*-pentoxychromone et de 7-*sec*-nonyloxychromone avec l'octen-3-ol

| | Indice de répulsion Dépôt 5mg | Indice de répulsion Dépôt 10 mg | Indice de répulsion Dépôt 30 mg |
|---|---|---|---|
| **7-*sec*-pentoxychromone** | -59,2% | -47,8% | -28,7% |
| **7-*sec*-nonyloxychromone** | -65,8% | -63,7% | -27,1% |
| **Octen-3-ol** | -41,7% | -54,7% | -61,6% |

L'analyse des résultats sur les alcoxy chromones montrent une activité attractante équivalente à celle de l'octénol. Cependant, il est connu que l'octénol a une tension de vapeur élevée contrairement aux alcoxy chromones qui pourront donc avoir un effet à plus longue durée.

Les résultats ci-dessus montrent que les composés de l'invention ont des propriétés attractantes ou répulsives importantes sur *Aedes albopictus* en fonction de la structure du substituant (longueur de la chaine, stéréochimie,...).

Contrairement aux groupements hydroxy ou méthoxy, les groupements 2-butoxy conduisent à des effets répulsifs importants et dépendant de la stéréochimie au niveau de la chaîne 2-butoxy. L'effet répulsif de l'énantiomère R est aussi important que le DEET et la picaridine à doses identiques.

Les groupements alkoxy secondaires supérieurs à butyle (pentyle et nonyle) conduisent à des produits de fortes masses moléculaires et possédant des propriétés attractantes. L'effet attractif dépend également de la stéréochimie du groupement 2 alkoxy. De même, les chromones avec des substituants alcoxy primaires à longues chaines (décyl par exemple) ont un effet attractif très élevé. Et en comparant avec l'attractif le plus utilisé (octénol) avec les mêmes doses, les alcoxychromones ont un effet attractif plus élevé.

Avec les 7-butoxychromones, il est possible de formuler des sprays, crèmes et textiles protecteurs contenant les produits seuls ou en mélange avec d'autres produits répulsifs.

Avec les produits attractifs, des pièges (mécaniques, matériaux imprégnés de colles,...) appâtés des produits seuls ou en mélange avec d'autres attractifs permettent un piégeage en masse des moustiques et d'autres insectes hématophages.

Ces produits dérivés du 7-hydroxychromone ont l'avantage d'avoir des masses moléculaires élevées leur permettant d'avoir une durée d'efficacité importante en termes de répulsion et d'attractivité vis-à-vis *d'Aedes albopictus.*

**Exemple 19 : Evaluation des propriétés répulsives dans un olfactomètre en cage**

L'évaluation longue durée (24 heures) est réalisée dans un système en cage (1m x 1m x 2m) équipé de deux pièges, un appâté et l'autre témoin. Les tests sont réalisés en parallèle dans deux salles d'expérimentation (3,5 m x 2m x 2m) qui sont maintenues à 25 +/- 5°C, humidité relative de 60%, pour une photopériode de 12 h (Andrianjafy *et al.* 2017). Dans la grande cage est placée une petite cage (35 cm× 35 cm× 35 cm) où sont placés les moustiques avant le lâcher ainsi que les deux pièges (avec le produit testé et le contrôle). Ces pièges sont séparés de 1,45 m. Pour les tests, 100 μl des solutions sont déposés sur des papiers filtres en utilisant l'éthanol comme solvant. L'éthanol est évaporé avant l'introduction dans le piège. Une source de $CO_2$ est placée à l'extérieur de la cage afin d'augmenter l'activité des moustiques. 25 femelles *Aedes albopictus* sont utilisées pour chaque test qui dure 24h et débute à 9h du matin. Six réplications sont réalisées pour chaque dose d'un produit.

> Test sur une durée de 24h en cage pour les propriétés répulsives du (*R*) 7-*sec*-butoxychromone.
> Test sur une durée de 24h en cage pour les propriétés attractives du (*RS*) 7-*sec*-nonyloxychromone.
> Test *in natura* avec un piège « sentinelle » du racémique (*RS*) 7-*sec*-nonyloxychromone.
> Test des effets synergiques pour les propriétés attractives de (*RS*) 7-*sec*-nonyloxychromone et de la 4-hydroxycoumarine

**Exemple 20 : Tests avec pièges**

Des tests « *in natura* » d'un composé ou d'un mélange selon l'invention sont réalisés avec le piège sentinelle BG (Biogent®) et le piège lumineux CDC pour capturer *Aedes* sp. et *Anopheles* sp., respectivement. Les résultats obtenus sont concluants.

**Exemple 21 : Evaluation de la combinaison d'un composé de l'invention avec un composé chromone**

Il est possible d'associer un composé chromone avec d'autres composés de structure différente. Par exemple la 4-hydroxy-coumarine est attractant sélectif *d'Aedes albopictus,* l'utilisation d'un mélange 50/50 de 4-hydroxy-coumarine et de 7-(2nonyloxy)-chromone a un pouvoir attractant supérieur aux deux composés utilisés séparément.

Le 7-nonyloxychromone a un effet attractif à faible dose (ou quantité) avec un indice de Kairomone IK=57%. Une diminution importante de cet effet est observée à partir de la dose 10 mg. De même, à faible dose, la 4-hydroxycoumarine a aussi un effet attractif avec un IK de l'ordre de 40%. Une augmentation de 10% d'attractivité a été enregistrée pour des doses plus élevées (10 et 30 mg).

Un effet synergique est observé entre la 4-hydroxycoumarine et le 7-nonyloxychromone (cf. Figure 1). Cet effet est généralement peu dépendant de la concentration, contrairement aux produits purs, avec un IK>55% pour toutes les doses testées, ce qui est un avantage pratique important. Nous supposons que la 4-hydroxycoumarine (aspect : solide) stabilise le 7-nonyloxychromone (aspect : liquide) afin d'engendrer un effet attractif plus important.

**Exemple 22 :** Evaluation de la 7-n-décyloxychromone et de la 7-(2'-ethyl)hexyloxychromone

L'évaluation des propriétés attractantes des 7-n-decyloxychromone et 7-(2'-ethyl hexyloxychromone a été réalisée dans un olfactomètre tunnel en faisant varier la quantité de produit à tester, selon le protocole décrit plus haut.

Les Figures 2 et 3 représentent les résultats ainsi obtenus.

## Revendications

1. Utilisation d'un composé de formule (I-1) suivante :

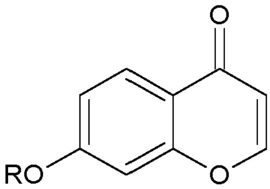

(I-1)

R représentant un groupe alkyle, linéaire ou ramifié, comprenant de 2 à 20 atomes de carbone,
ledit composé étant achiral lorsque R est un groupe linéaire ou sous forme d'un mélange racémique, d'un énantiomère (*R*) ou d'un énantiomère (*S*) lorsque R est un groupe ramifié,
comme attractif ou répulsif pour les invertébrés piqueurs hématophages, de préférence pour les moustiques, notamment des genres *Aedes*, *Anopheles* et *Culex,* ou encore les ceratopogonidés, les phlébotomes, les punaises, les puces et les tiques.

2. Utilisation selon la revendication 1, d'un composé de formule (II) :

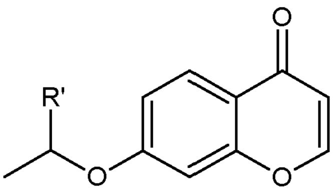

(II)

dans laquelle R' représente un groupe alkyle comprenant de 3 à 18 atomes de carbone, comme attractif pour les invertébrés piqueurs hématophages.

**3.** Utilisation selon la revendication 1, d'un composé de formule (II) :

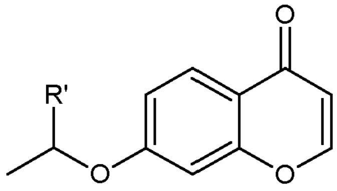

(II)

dans laquelle R' représente un groupe alkyle comprenant 2 atomes de carbone, comme répulsif pour les invertébrés piqueurs hématophages.

**4.** Utilisation selon la revendication 3, dans laquelle le composé de formule (II) est sous forme de l'énantiomère (*R*) ou d'un mélange racémique, ou dans laquelle le composé de formule (II) est sous forme de l'énantiomère (*S*), et dans laquelle le composé est utilisé dans une composition dans laquelle la teneur dudit composé est supérieure ou égale à 20% en poids par rapport au poids total de ladite composition.

**5.** Utilisation selon la revendication 2, d'un composé de formule (II) comme attractif pour les invertébrés piqueurs hématophages, dans laquelle R' représente un groupe alkyle comprenant 2 atomes de carbone, ledit composé étant sous forme de l'énantiomère (*S*), et dans laquelle le composé est utilisé dans une composition dans laquelle la teneur dudit composé est inférieure ou égale à 20% en poids par rapport au poids total de ladite composition.

**6.** Composition attractive ou répulsive pour des invertébrés piqueurs hématophages, comprenant au moins un composé de formule (I-1) suivante :

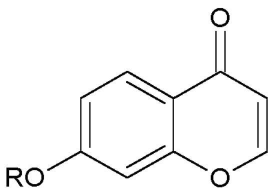

(I-1)

R représentant un groupe alkyle, linéaire ou ramifié, comprenant de 2 à 20 atomes de carbone,
ledit composé étant achiral lorsque R est un groupe linéaire ou sous forme d'un mélange racémique, d'un énantiomère (*R*) ou d'un énantiomère (*S*) lorsque R est un groupe ramifié,
ladite composition comprenant éventuellement en outre un composé additionnel attractif ou répulsif pour un invertébré piqueur hématophage.

**7.** Composition attractive pour des invertébrés piqueurs hématophages selon la revendication 6, comprenant:

- au moins un composé de formule (I-1), dans laquelle R représente un groupe alkyle comprenant de 2 à 16 atomes de carbone, ou
- au moins un composé de formule (I-1), dans laquelle R représente un groupe alkyle comprenant 4 atomes de carbone, ledit composé étant sous forme de l'énantiomère (S), et dans laquelle le composé sous forme de l'énantiomère (S) est utilisé en une teneur inférieure ou égale à 20% de ladite composition.

**8.** Composition répulsive pour des invertébrés piqueurs hématophages selon la revendication 6, comprenant:

- au moins un composé de formule (I-1) dans laquelle R représente un groupe alkyle comprenant 4 atomes de carbone, le composé de formule (I-1) étant sous forme de l'énantiomère (R) ou d'un mélange racémique, ou d'un mélange des énantiomères (R) et (S) en des teneurs massiques différentes de 50/50% ; ou
- au moins un composé de formule (I-1) dans laquelle R représente un groupe alkyle comprenant 4 atomes de carbone, ledit composé étant sous forme de l'énantiomère (S) et dans laquelle ledit composé sous forme de

l'énantiomère (S) est utilisé en une teneur supérieure ou égale à 20% en poids par rapport au poids de ladite composition.

ladite composition étant notamment sous forme de spray ou de crème, de support ou de cartouches pour diffuseurs.

**9.** Kit pour le piégeage d'un invertébré piqueur hématophage, notamment *Aedes albopictus,* comprenant :
au moins une composition attractive comprenant au moins un composé de formule (I-1) suivante :

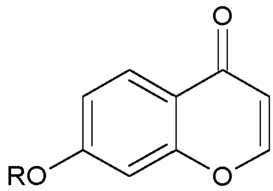
(I-1)

R représentant un groupe alkyle, linéaire ou ramifié, comprenant de 2 à 20 atomes de carbone, ledit composé étant achiral lorsque R est un groupe linéaire ou sous forme d'un mélange racémique, d'un énantiomère (*R*) ou d'un énantiomère (*S*) lorsque R est un groupe ramifié,

- ladite composition comprenant éventuellement en outre un composé additionnel attractif pour un invertébré piqueur hématophage , et

au moins un piège pour invertébré piqueur hématophage, notamment *Aedes albopictus.*

**10.** Kit pour le piégeage d'un invertébré piqueur hématophage selon la revendication 9, dans lequel la composition attractive comprend :

- au moins un composé de formule (I-1), dans laquelle R représente un groupe alkyle comprenant de 2 à 16 atomes de carbone, ou
- au moins un composé de formule (I-1), dans laquelle R représente un groupe alkyle comprenant 4 atomes de carbone, ledit composé étant sous forme de l'énantiomère (S), et dans laquelle le composé sous forme de l'énantiomère (S) est utilisé en une teneur inférieure ou égale à 20% de ladite composition.

**11.** Utilisation du kit selon les revendications 9 ou 10, pour le piégeage d'invertébrés piqueurs hématophages, notamment de moustiques, en particulier *Aedes albopictus* et *Anopheles sp.*

**12.** Support répulsif pour les invertébrés piqueurs hématophages, notamment *Aedes albopictus,* ledit support étant imprégné d'au moins une composition répulsive comprenant:

- au moins un composé de formule (I-1) suivante :

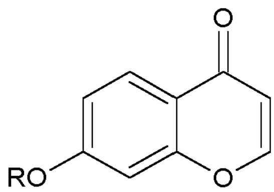
(I-1)

dans laquelle R représente un groupe alkyle comprenant 4 atomes de carbone, le composé de formule (I-1) étant sous forme de l'énantiomère (*R*) ou d'un mélange racémique, ou d'un mélange des énantiomères (*R*) et (*S*) en des teneurs massiques différentes de 50/50% ; ou
- au moins un composé de formule (I-1) dans laquelle R représente un groupe alkyle comprenant 4 atomes de carbone, ledit composé étant sous forme de l'énantiomère (*S*) et dans laquelle ledit composé sous forme de l'énantiomère (*S*) est utilisé en une teneur supérieure ou égale à 20% en poids par rapport au poids de ladite composition, ladite composition comprenant éventuellement en outre un composé additionnel répulsif pour un invertébré piqueur hématophage, ledit support étant notamment choisi dans le groupe constitué des textiles,

notamment les moustiquaires, des vêtements pour randonnées, des bracelets, colliers ou cartouches pour diffuseurs.

## Patentansprüche

1. Verwendung einer Verbindung mit folgender Formel (I-1):

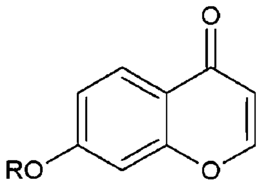

(I-1)

wobei R eine lineare oder verzweigte Alkylgruppe darstellt, die 2 bis 20 Kohlenstoffatome umfasst,
wobei die Verbindung achiral ist, wenn R eine lineare Gruppe ist oder in Form eines Racemats, eines (R)-Enantiomers oder eines (S)-Enantiomers vorliegt, wenn R eine verzweigte Gruppe ist,
als Lockmittel oder Abschreckmittel für blutsaugende stechende Wirbellose, vorzugsweise für Mücken, insbesondere der Gattungen *Aedes, Anopheles* und *Culex,* oder auch für Keratopogonide, Phlebotome, Wanzen, Flöhe und Zecken.

2. Verwendung nach Anspruch 1 einer Verbindung der Formel (II):

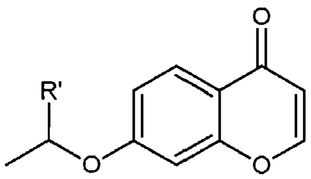

(II)

wobei R' eine Alkylgruppe darstellt, die 3 bis 18 Kohlenstoffatome umfasst, als Lockmittel für blutsaugende stechende Wirbellose.

3. Verwendung nach Anspruch 1 einer Verbindung der Formel (II):

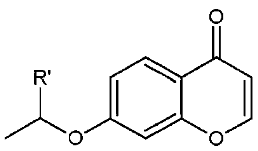

(II)

wobei R' eine Alkylgruppe darstellt, die 2 Kohlenstoffatome umfasst, als Abschreckmittel für blutsaugende stechende Wirbellose.

4. Verwendung nach Anspruch 3, wobei die Verbindung der Formel (II) in Form des (*R*)-Enantiomers oder eines Racemats vorliegt, oder wobei die Verbindung der Formel (II) in Form des (*S*)-Enantiomers vorliegt, und wobei die Verbindung in einer Zusammensetzung verwendet wird, in der der Gehalt der Verbindung größer oder gleich 20 Gew.-%, bezogen auf das Gesamtgewicht der Zusammensetzung, ist.

5. Verwendung nach Anspruch 2 einer Verbindung der Formel (II) als Lockmittel für blutsaugende stechende Wirbellose, wobei R' eine Alkylgruppe darstellt, die 2 Kohlenstoffatome umfasst, wobei die Verbindung in Form des (S)-Enantiomers vorliegt, und wobei die Verbindung in einer Zusammensetzung verwendet wird, in der der Gehalt der Verbindung kleiner oder gleich 20 Gew.-%, bezogen auf das Gesamtgewicht der Zusammensetzung, ist.

6. Zusammensetzung, die für blutsaugende stechende Wirbellose anlockend oder abschreckend ist, umfassend mindestens eine Verbindung mit folgender Formel (I-1):

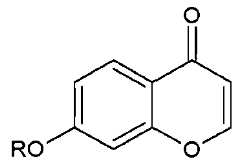
(I-1)

wobei R eine lineare oder verzweigte Alkylgruppe darstellt, die 2 bis 20 Kohlenstoffatome umfasst,
wobei die Verbindung achiral ist, wenn R eine lineare Gruppe ist oder in Form eines Racemats, eines (*R*)-Enantiomers oder eines (*S*)-Enantiomers vorliegt, wenn R eine verzweigte Gruppe ist,
wobei die Zusammensetzung gegebenenfalls ferner eine zusätzliche Verbindung umfasst, die für einen blutsaugenden stechenden Wirbellosen anlockend oder abschreckend ist.

**7.** Zusammensetzung, die für blutsaugende stechende Wirbellose anlockend ist, nach Anspruch 6, umfassend:

- mindestens eine Verbindung der Formel (I -1), in welcher R eine Alkylgruppe darstellt, die 2 bis 16 Kohlenstoffatome umfasst, oder
- mindestens eine Verbindung der Formel (I -1), in welcher R eine Alkylgruppe darstellt, die 4 Kohlenstoffatome umfasst, wobei die Verbindung in Form des (S)-Enantiomers vorliegt, und wobei die Verbindung in Form des (S)-Enantiomers in einem Gehalt kleiner oder gleich 20 % der Zusammensetzung verwendet wird.

**8.** Zusammensetzung, die für blutsaugende stechende Wirbellose abschreckend ist, nach Anspruch 6, umfassend:

- mindestens eine Verbindung der Formel (I-1), wobei R eine Alkylgruppe darstellt, die 4 Kohlenstoffatome umfasst, wobei die Verbindung der Formel (I-1) in Form des (*R*)-Enantiomers oder eines Racemats oder eines Gemischs der (*R*)- und (*S*)-Enantiomere in von 50/50 % unterschiedlichen Massengehalten vorliegt; oder
- mindestens eine Verbindung der Formel (I-1), wobei R eine Alkylgruppe darstellt, die 4 Kohlenstoffatome umfasst, wobei die Verbindung in Form des (*S*)-Enantiomers vorliegt und wobei die Verbindung in Form des (*S*)-Enantiomers in einem Gehalt größer oder gleich 20%, bezogen auf das Gewicht der Zusammensetzung, verwendet wird,

wobei die Zusammensetzung insbesondere in Form von Spray oder Creme, Träger oder Kartuschen für Zerstäuber vorliegt.

**9.** Set zum Einfangen eines blutsaugenden stechenden Wirbellosen, insbesondere von *Aedes albopictus,* umfassend: mindestens eine anlockende Zusammensetzung, die mindestens eine Verbindung mit folgender Formel (I-1) umfasst:

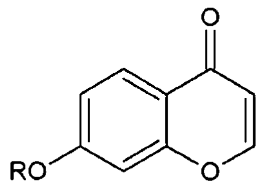
(I-1)

wobei R eine lineare oder verzweigte Alkylgruppe darstellt, die 2 bis 20 Kohlenstoffatome umfasst,
wobei die Verbindung achiral ist, wenn R eine lineare Gruppe ist oder in Form eines Racemats, eines (*R*)-Enantiomers oder eines (*S*)-Enantiomers vorliegt, wenn R eine verzweigte Gruppe ist,

- wobei die Zusammensetzung gegebenenfalls ferner eine zusätzliche Verbindung umfasst, die für einen blutsaugenden stechenden Wirbellosen anlockend ist, und

mindestens eine Falle für blutsaugende stechende Wirbellose, insbesondere *Aedes albopictus.*

**10.** Set zum Einfangen eines blutsaugenden stechenden Wirbellosen nach Anspruch 9, wobei die anlockende Zusammensetzung umfasst:

- mindestens eine Verbindung der Formel (I -1), in welcher R eine Alkylgruppe darstellt, die 2 bis 16 Kohlenstoffatome umfasst, oder
- mindestens eine Verbindung der Formel (I -1), wobei R eine Alkylgruppe darstellt, die 4 Kohlenstoffatome umfasst, wobei die Verbindung in Form des (S)-Enantiomers vorliegt, und wobei die Verbindung in Form des (S)-Enantiomers in einem Gehalt kleiner oder gleich 20 % der Zusammensetzung verwendet wird.

**11.** Verwendung des Sets nach den Ansprüchen 9 oder 10 zum Einfangen von blutsaugenden stechenden Wirbellosen, insbesondere von Mücken, vor allem von *Aedes albopictus* und *Anopheles sp.*

**12.** Träger, der für blutsaugende stechende Wirbellose abschreckend ist, insbesondere für *Aedes albopictus,* wobei der Träger mit mindestens einer abschreckenden Zusammensetzung imprägniert ist, umfassend:

- mindestens eine Verbindung mit folgender Formel (I-1):

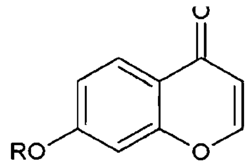
(I-1)

wobei R eine Alkylgruppe darstellt, die 4 Kohlenstoffatome umfasst, wobei die Verbindung der Formel (I-1) in Form des (*R*)-Enantiomers oder eines Racemats oder eines Gemischs der (*R*)- und (*S*)-Enantiomere in von 50/50 % unterschiedlichen Massengehalten vorliegt; oder
- mindestens eine Verbindung der Formel (I -1), wobei R eine Alkylgruppe darstellt, die 4 Kohlenstoffatome umfasst, wobei die Verbindung in Form des (*S*)-Enantiomers vorliegt und wobei die Verbindung in Form des (*S*)-Enantiomers in einem Gehalt größer oder gleich 20 Gew.-%, bezogen auf das Gewicht der Zusammensetzung, verwendet wird, wobei die Zusammensetzung gegebenenfalls ferner eine zusätzliche Verbindung umfasst, die für einen blutsaugenden stechenden Wirbellosen abschreckend ist, wobei der Träger insbesondere aus der Gruppe ausgewählt wird, die aus Textilien, insbesondere Insektenschutzgittern, Wanderbekleidung, Armbändern, Halsketten oder Kartuschen für Zerstäuber besteht.

**Claims**

**1.** The use of a compound with formula (I-1):

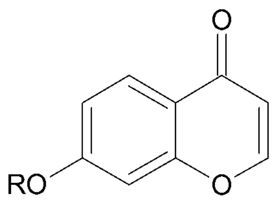
(I-1)

R being a linear or branched alkyl group comprising from 2 to 20 carbon atoms,
said compound being achiral when R is a linear group or in the form of a racemic mixture, an enantiomer (R) or an enantiomer (S) when R is a branched group,
as an attractant or repellent for blood-sucking, biting invertebrates, preferentially for mosquitoes, in particular of the genera Aedes, Anopheles and Culex, or alternatively ceratopogonidae, phlebotominae, bugs, fleas and ticks.

**2.** The use according to claim 1 of a compound with the formula (II):

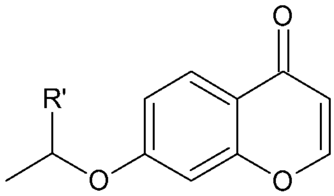

(II)

wherein R' represents an alkyl group containing 3 to 18 carbon atoms, as an attractant for blood-sucking, biting invertebrates.

**3.** The use according to claim 1 of a compound with the formula (II):

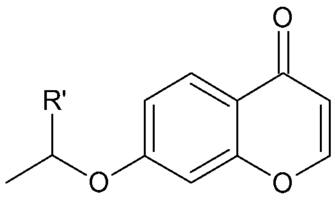

(II)

wherein R' represents an alkyl group containing 2 carbon atoms, as a repellent for blood-sucking, biting invertebrates.

**4.** The use according to claim 3, wherein the compound with the formula (II) is in the form of the enantiomer (R) or a racemic mixture, or wherein the compound with the formula (II) is in the form of the enantiomer (S), and wherein the compound is used in a composition wherein the content of said compound is greater than or equal to 20% by weight with respect to the total weight of said composition.

**5.** The use according to claim 2 of a compound with the formula (II) as attractant for blood-sucking, biting invertebrates, wherein R' represents an alkyl group comprising 2 carbon atoms, said compound being in the form of the enantiomer (S), and wherein the compound is used in a composition wherein the content of said compound is less than or equal to 20% by weight with respect to the total weight of said composition.

**6.** An attractant or repellent composition for blood-sucking, biting invertebrates comprising at least one compound of the following formula (I-1):

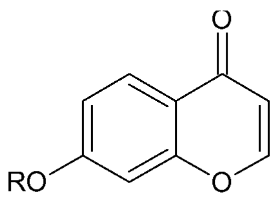

(I-1)

R being a linear or branched alkyl group comprising from 2 to 20 carbon atoms,
said compound being achiral when R is a linear group or in the form of a racemic mixture, an enantiomer (R) or an enantiomer (S) when R is a branched group,
said composition optionally further comprising an additional compound which is attractant or repellent for a blood-sucking, biting invertebrate.

**7.** The attractant composition for blood-sucking, biting invertebrates according to claim 6, comprising:

- at least one compound with the formula (I-1), wherein R represents an alkyl group comprising from 2 to 16 carbon atoms, or
- at least one compound with the formula (I-1), wherein R represents an alkyl group comprising 4 carbon atoms, said compound being in the form of the enantiomer (S), and wherein the compound in the form of the enantiomer (S) is used in a concentration smaller than or equal to 20% of said composition.

**8.** The repellent composition for blood-sucking, biting invertebrates according to claim 6, comprising:

- at least one compound with the formula (I-1) wherein R represents an alkyl group containing 4 carbon atoms, the compound with the formula (I-1) being in the form of the enantiomer (R) or of a racemic mixture, or of a mixture of the enantiomers (R) and (S) in mass concentrations different from 50/50%; or
- at least one compound with the formula (I-1) wherein R represents an alkyl group comprising 4 carbon atoms, said compound being in the form of the enantiomer (S) and wherein said compound in the form of the enantiomer (S) is used in a concentration greater than or equal to 20% by weight with respect to the weight of said composition,

said composition being in particular in the form of a spray or cream, a support or cartridges for diffusers.

**9.** A kit for trapping blood-sucking, biting invertebrate, in particular *Aedes albopictus,* comprising:
at least one attractant composition comprising at least one compound of the following formula (I-1):

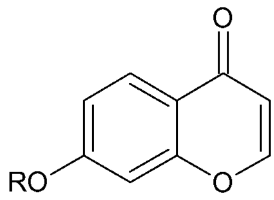

(I-1)

R being a linear or branched alkyl group comprising from 2 to 20 carbon atoms,
said compound being achiral when R is a linear group or in the form of a racemic mixture, an enantiomer (R) or an enantiomer (S) when R is a branched group,

- said composition optionally further comprising an additional compound which is attractant for a blood-sucking, biting invertebrate, and
at least one blood-sucking, biting invertebrate trap, in particular *Aedes albopictus.*

**10.** A kit for trapping blood-sucking, biting invertebrate according to claim 9, wherein the attractant composition comprises:

- at least one compound with the formula (I-1), wherein R represents an alkyl group comprising from 2 to 16 carbon atoms, or
- at least one compound with the formula (I-1), wherein R represents an alkyl group comprising 4 carbon atoms, said compound being in the form of the enantiomer (S), and wherein the compound in the form of the enantiomer (S) is used in a concentration smaller than or equal to 20% of said composition.

**11.** The use of the kit according to claims 9 or 10 for trapping blood-sucking, biting invertebrates, in particular mosquitoes, more particularly *Aedes albopictus* and *Anopheles sp.*

**12.** A repellent support for blood-sucking, biting invertebrates, in particular Aedes *albopictus,* said support being impregnated with at least one repellent composition comprising:

- at least one compound with the following formula (I-1):

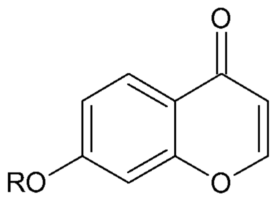

(I-1)

wherein R represents an alkyl group containing 4 carbon atoms, the compound with the formula (I-1) being in the form of the enantiomer (R) or of a racemic mixture, or of a mixture of the enantiomers (R) and (S) in mass

concentrations different from 50/50%; or
- at least one compound with the formula (I-1) wherein R represents an alkyl group comprising 4 carbon atoms, said compound being in the form of the enantiomer (S) and wherein said compound in the form of the enantiomer (S) is used in a concentration greater than or equal to 20% by weight with respect to the weight of said composition, said composition optionally further comprising an additional compound which is attractant for a blood-sucking, biting invertebrate, said support being chosen in particular from the group consisting of textiles, in particular mosquito nets, clothing for hiking, bracelets, necklaces or cartridges for diffusers.

FIG.1

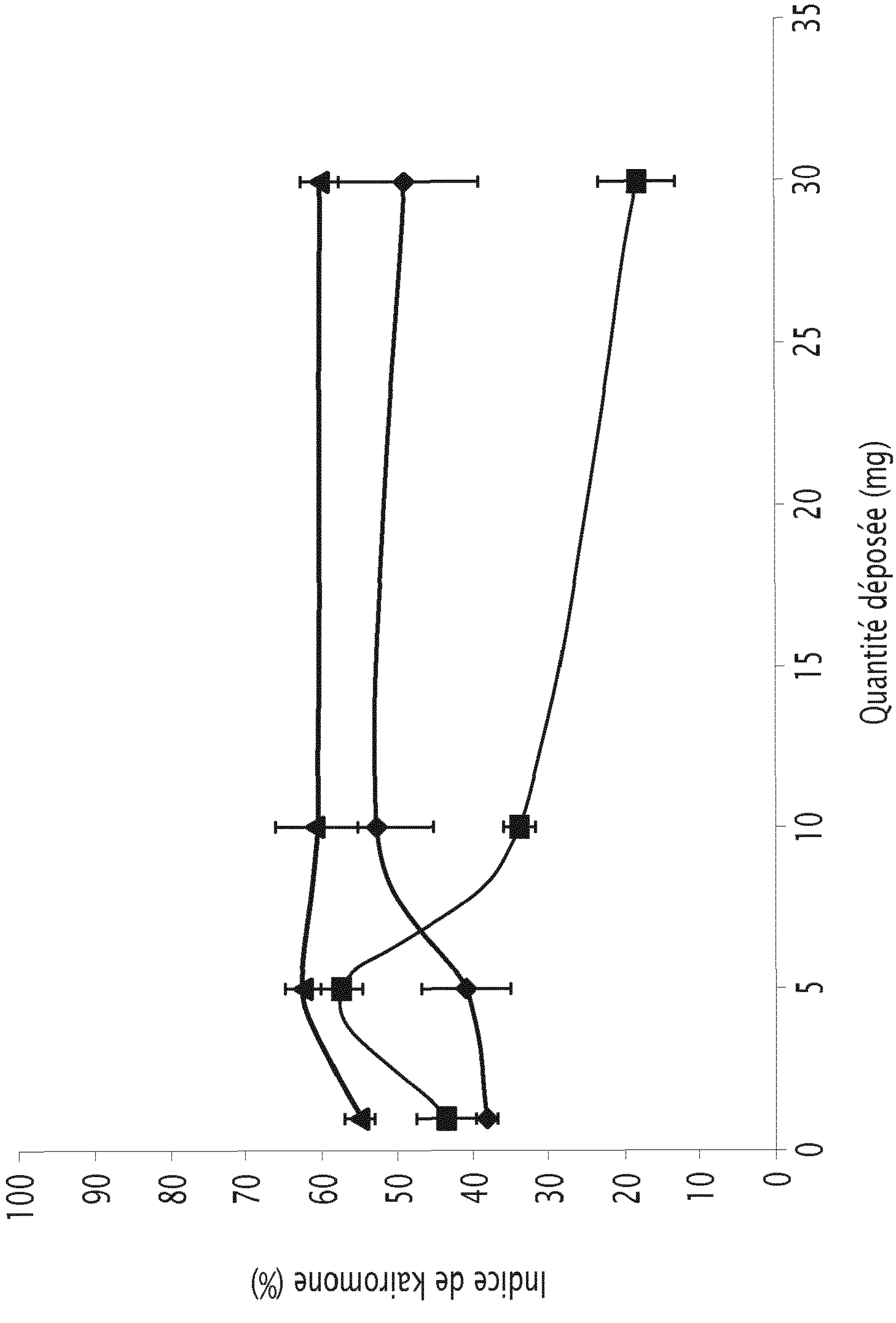
Indice de kairomone (%)
100
90
80
70
60
50
40
30
20
10
0
0
5
10
15
20
25
30
35
Quantité déposée (mg)

FIG.2

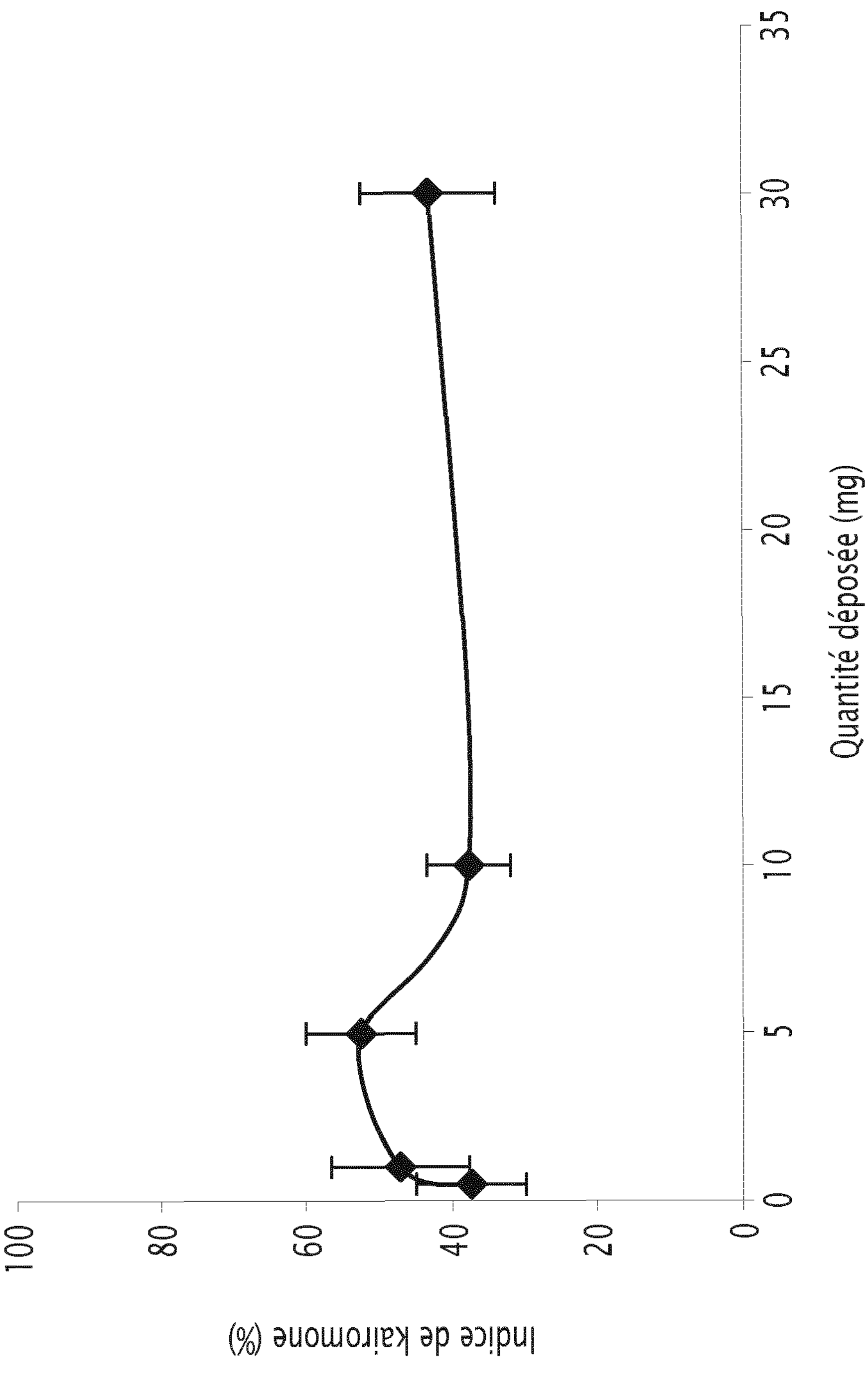
Indice de kairomone (%)
100
80
60
40
20
0
Quantité déposée (mg)
0
5
10
15
20
25
30
35

FIG.3

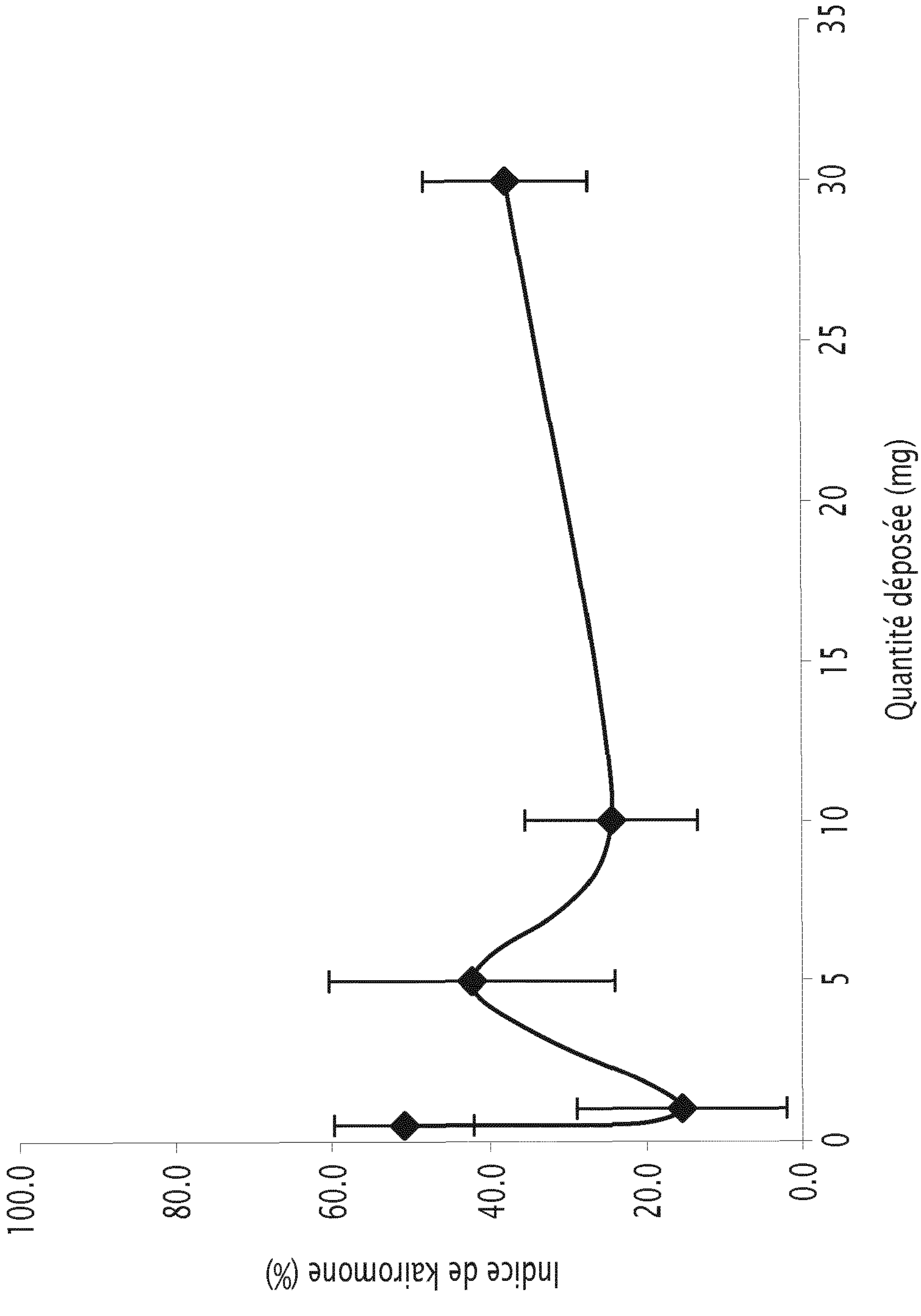
Indice de kairomone (%)
100.0
80.0
60.0
40.0
20.0
0.0
0
5
10
15
20
25
30
35
Quantité déposée (mg)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2012329832 A


### Littérature non-brevet citée dans la description


- *Thèse Andrianjafy.*, 2018, vol. 119, 120p
- *Thèse Andrianjafy*, 2018, 120-121p
- **ANDRIANJAFY MT** ; **RAVAOMANARIVO LH** ; **VESTALYS RAMANANDRAIBE V** ; **RAKOTOMANGA MF** ; **MAVINGUI P** ; **LEMAIRE M**. Synthesis, bioassays and field evaluation of hydroxycoumarins and their alkyl derivatives as repellents or kairomones for Aedes albopictus Skuse (Diptera: Culicidae). *J Chem Ecol*, 2018, vol. 44 (3), 299-311
- **BECKER NM** ; **ZGOMBA DP** ; **LUDWIG M**. Comparison of carbon dioxide, octanol and o a houst-odour as mosquito attractants in the Upper Valley. *Germany. Med Vet Entomol*, 1995, vol. 9, 377-380
- **HALL DR** ; **BEEVOR PS** ; **CORK A** ; **NESBITT BF** ; **VALE GA**. 1-octen-ol: a potent olfactory stimulant and attractant for tsetse isolated from cattle odours.. *Insect Sci Appl*, 1984, vol. 5, 335-339
- **SHONE SM** ; **FERRAO PN** ; **LESSER CR** ; **GLASS GE** ; **NORRIS DE**. Evaluation of carbon dioxide and 1-octen-3-ol-baited centers for disease control Fay-Prince traps to collect Aedes albopictus. *J Am Mosq Control Assoc*, 2003, vol. 19, 445-447
- **GOVERE J** ; **DURRHEIM DN** ; **DU TOIT N** ; **HUNT RH**. Local plants as repellents against Anopheles arabiensis, in Mpumalanga Province, South Africa. *Cent Afr J Med*, 2000, vol. 46 (8), 213-6
- **BARNARD DR**. Laboratory evaluation of mosquito repellents against Aedes albopictus, Culex nigripalpus, and Ochlerotatus triseriatus (Diptera: Culicidae).. *Journal of Med Entomol*, 2004, vol. 41, 726-730